# EUROPEAN PATENT SPECIFICATION

(11) **EP 3 777 931 B1**
(45) Date of publication and mention of the grant of the patent: **02.07.2025**
(21) Application number: 19777806.1
(22) Date of filing: 27.03.2019
(51) Int. Cl.: A61M 5/28, A61M 5/178, A61J 1/20

(54) **PREFILLED TWO-COMPONENT-MIXING SYRINGE KIT**
VORGEFÜLLTES ZWEIKOMPONENTEN-MISCHSPRITZENKIT
KIT SERINGUE DE MÉLANGE DE DEUX COMPOSANTS PRÉ-REMPLI

(30) Priority: 28.03.2018 JP 2018062038
(43) Date of publication of application: 17.02.2021
(73) Proprietor: CMC-Pharma Co., Ltd, Osaka-shi, Osaka, 555-0012 (JP)
(72) Inventor: MORIMOTO, Shuji, Suita-shi, Osaka 565-0843 (JP); YAMAUTI, Hirosi, Kawanishi-shi, Hyogo 666-0112 (JP); KISHIMOTO, Jyotaro, Nishinomiya-shi, Hyogo 662-0895 (JP)
(74) Representative: Dennemeyer & Associates S.A.
(86) International application number: PCT/JP2019/013354
(87) International publication number: WO 2019/189457

(56) References cited:
- FR-A1- 2 708 469
- JP-A- 2012 010 930
- JP-A- 2019 058 311
- JP-A- H09 502 116
- JP-A- H11 502 731
- US-A- 5 088 996
- US-A- 5 125 908

## Description

### Technical field

This invention relates to a two-component-mixing prefilled syringe kit in which a syringe barrel filled with one component constituting an injection solution and a container filled with the other component of the injection solution are arranged to face each other, and the component in the syringe barrel and the component in the container are mixed to prepare the injection solution.

### Background art

According to Patent document 1 related to a conventional prefilled syringe kit, a structure is known in which a liquid component is filled inside a syringe barrel, and an injection needle attached to the tip of the syringe barrel is arranged to face and be separated from a rubber seal member at the outlet of a container filled with a drug, and the syringe barrel and the drug container are connected by a nesting mechanism so as to be relatively movable in the barrel axis direction of the syringe barrel. In Patent document 1, during use, the syringe barrel is pushed toward the drug container side, the tip of the injection needle is pierced through the seal member, the inside of the syringe barrel and the inside of the drug container are communicated, and then a plunger inserted into the rear end opening of the syringe barrel is pushed to inject the liquid component inside the syringe barrel into the drug container to mix the drug and the liquid, and a prepared injection solution is returned to the inside of the syringe barrel.

Also, according to another Patent document 2, a structure is known in which a powder drug is filled in a syringe barrel, and a tip of an injection needle attached to a tip of the syringe barrel is arranged to face and be pierced (but, in non-penetrating manner) by a seal member at an outlet of a container filled with a liquid component, and the syringe barrel and the liquid container are connected so as to be relatively movable in the axial direction of the syringe barrel by a connection barrel. In the prefilled syringe kit of Patent document 2, during use, a plunger inserted into the rear end opening of the syringe barrel is pushed to allow the tip of the injection needle to penetrate the seal member, thereby the inside of the syringe barrel and the inside of the liquid container are communicated, pressurized air is sent to the inside of the liquid container, and the liquid component in the container is moved to the syringe barrel side by the pressurized air to mix the drug and the liquid component to prepare an injection solution. Patent document 3 discloses a fluid tight holding chamber which accumulates solution from the vial that aspirates during mixing or drawing fluid from the vial or is pressured out of the syringe upon extraction of the cannula from the vial, and Patent document 4 discloses hypodermic syringe employs a syringe holder which surrounds the syringe barrel, including the needle, both before and after injection.

### Preceding technical documents

### Patent documents

Patent document 1: JP S59-118164 A
Patent document 2: JP H05-31189 A
Patent document 3: US 5088996 A
Patent document 4: US 5125908 A

### Summary of the invention

### Problems to be resolved by the invention

In the prefilled syringe kit described in Patent document 1, the syringe barrel, the plunger, the drug container and the like are exposed to the outside. In the prefilled syringe kit described in Patent document 2, the syringe barrel and the plunger are covered by a cover attached to the rear edge of the connecting barrel, but the liquid container, the connecting portion between the connecting barrel and the liquid container, and the like are exposed to the outside. Therefore, in the conventional prefilled syringe kit, during storage and preparation of the injection solution, there is a possibility that the drug component, the liquid component, or the like filled inside leaks to the outside. In particular, in the case that the injection needle penetrates the seal member of the liquid container to transfer the liquid inside the container to the side of the syringe barrel and mix it with the component in the syringe barrel to prepare the injection solution, after the preparation of the injection solution, when the injection needle is pulled out from the container, the liquid component remaining in the container may leak to the outside through the needle piercing point of the seal member.

In view of the above problems, an object of the present invention is to provide a two-component-mixing prefilled syringe kit in which a leak of the liquid component remaining in the container to the outside can be effectively prevented when preparing the injection solution by mixing the drug component in the syringe barrel and the liquid component in the container.

### Means of solving the problems

A two-component-mixing prefilled syringe kit according to the present invention comprises; a syringe barrel in which an injection needle is attached to one end, a plunger is slidably inserted from an opening on the other end, and a solid component or a liquid component constituting an injection solution is filled inside; a container in which an outlet is sealed with a seal member that can be pierced by the injection needle, and a liquid component constituting the injection solution is filled inside; and a holding member for holding at least one of the syringe barrel or the container so as to be movable along the barrel axis direction of the syringe barrel with a tip of the injection needle facing the outlet of the container; wherein, it is characterized that a space-forming body which forms a space facing a surface of the seal member with a space sealed from the outside is arranged so as to be in close contact with the surface of the seal member, and the space-forming body is formed of a material that can be pierced by the injection needle at a portion facing the tip of the injection needle along the barrel axis direction of the injection barrel.

The two-component-mixing prefilled syringe kit according to the present invention comprises a needle cap which is formed of a material that can be pierced by the injection needle to cover the injection needle from the tip side, and fixed at the base end side to the base of the injection needle or the outer surface of the syringe barrel, and the needle cap has a tubular portion in which a tube axis direction is along to the direction toward the seal member and an expected piercing point by the injection needle is located at the inner side, on a front portion facing the seal member, and the space-forming body is constituted by the front portion of the needle cap and the tubular portion in close contact with the surface of the seal member.

The two-component-mixing prefilled syringe kit according to the present invention is characterized that the needle cap has a flange portion being in a direction intersecting with the barrel axis direction of the syringe barrel on an outer side surface of the front portion, and the holding member has a mount portion for mounting the syringe barrel, a pushing portion for pushing the flange portion against the container when moving to the container side along the barrel axis direction of the syringe barrel, and a lock part for holding the state where the pushing portion is pushing the flange portion against the container.

In the present invention, at least one of the syringe barrel or the container held by the holding member with the tip of the injection needle facing the outlet of the container moves along the barrel axis direction of the syringe barrel so that the container and the syringe barrel approach each other, and the tip of the injection needle attached to one end of the syringe barrel pierces the portion of the space-forming body and the seal member of the outlet of the container that face along the barrel axis direction, and after the inside of the container and the inside of the syringe barrel have communicated, the plunger slidably inserted from the other end opening of the syringe barrel is operated to push the air in the syringe barrel into the container inside, then the liquid component in the container is moved into the syringe barrel by the pressurized air in the container, mixing with the solid component or liquid component in the syringe barrel so as to prepare the injection solution. The injection needle pierced with the seal member of the container is pulled out from the seal member in order to separate the syringe barrel containing the prepared injection solution from the container. At this time, the liquid component remaining in the container may leak to the surface of the seal member from a needle piercing point of the seal member which the injection needle has got out, but the liquid component does not leak outside because the surface of the seal member is exposed to the space sealed from the outside.

In the present invention, when the container and the syringe barrel are approached each other and the tubular portion attached on the front portion of the needle cap facing the seal member of the container is pushed against the seal member, a space which the surface of the seal member faces is formed of a space sealed from the outside by the front portion and the tubular portion of the needle cap. Furthermore, the tip of the injection needle is passed through the inside of the tubular portion from the expected piercing point at the front portion of the needle cap to pierce the seal member of the container outlet, the inside of the container and the inside of the syringe barrel communicate, then moving the liquid component in the container into the syringe barrel, the injection solution is prepared. When pulling out the injection needle pierced with the seal member of the container from the seal member in order to separate the syringe barrel containing the prepared injection solution from the container, the liquid component in the container may leaks to the surface of the seal member, but since the space which the surface of the seal member faces is sealed from the outside by the front portion of the needle cap and the tubular portion pushed against the seal member, the liquid component does not leak to the outside.

In the present invention, the holding member with the syringe barrel mounted on the mount portion is moved toward the container side along the barrel axis direction so that the tip of the injection needle pierces the front portion of the needle cap and the seal member of the container, and when the flange portion on the outer side surface of the front portion of the needle cap intersecting with the barrel axis direction of the syringe barrel is pushed against the container by the pushing portion provided on the holding member, the state where the pushing portion is pushing the flange portion against the container is held by the lock part provided on the holding member. As a result, the tip of the injection needle pierces the front portion of the needle cap and the seal member of the container, and by the front portion of the needle cap and the tubular portion pushed against the seal member, the state that the surface of the seal member faces to the space sealed from the outside is stably maintained.

Furthermore, when the syringe barrel containing the prepared injection solution is taken out to the outside along the barrel axis direction, as the flange portion of the needle cap is pushed against the container side to be clamped by the pushing portion of the holding member, the needle cap fixed at the base end side to the base of the injection needle or the outer surface of the syringe barrel is forcibly separated from the syringe barrel and remains inside of the prefilled syringe kit, and the syringe with the needle cap removed can be taken out to the outside. As a result, it is not necessary to remove the needle cap when using the syringe.

A volume of the space surrounded by the surface of the seal member and the space-forming body is in the range of 0.001 ml to 0.03 ml.

Because, if it is less than 0.001 ml, there is a possibility that the amount of liquid leaking from the surface of the seal member may exceed 0.001 ml, while if it is more than 0.03 ml, the size of the space-forming body becomes unnecessarily large.

The length of the tubular portion along the tubular axis direction is less than 4 mm.

Because, if it is 4 mm or more, the moving distance required for the injection needle to pass front portion of the needle cap through the inside of the tubular portion to pierce the seal member of the container becomes long, and the operability is deteriorated.

The two-component-mixing prefilled syringe kit according to the present invention is characterized that; it comprises a needle cap which is formed of a material that can be pierced by the injection needle to cover the injection needle from the tip side, and fixed at the base end side to the base of the injection needle or the outer surface of the syringe barrel, and a tubular container adapter which has a tube axis coinciding with the barrel axis direction of the syringe barrel and fixed in close contact with the surface of the seal member at an one end of the tube axis direction, the needle cap has an insertion portion inserted along the tube axis direction of the container adapter from the other end side in the tube axis direction so as to be in close contact with the inner surface of the container adapter at a front portion facing the seal member, the space-forming body is constituted by the container adapter and the insertion portion of the needle cap.

In the present invention, inserting the insertion portion provided at the front portion of the needle cap along the tube axis direction from the other end side in the tube axis direction to the tubular container adapter that one end in the tube axis direction is fixed in close contact with the surface of the seal member, and the insertion portion is in close contact with the inner surface of the container adapter, the space facing the surface of the seal member is formed of a space sealed from the outside. The container and the syringe barrel approach each other, and the tip of the injection needle pierces the expected piercing place in the insertion portion of the needle cap and the seal member at the outlet of the container to communicate the inside of the container with the inside of the syringe barrel, and the liquid component in the container is moved into the syringe barrel to prepare an injection solution. When pulling out the injection needle that has pierced the seal member of the container from the seal member in order to separate the syringe barrel containing the prepared injection solution from the container, the liquid component remaining in the container may leaks to the surface of the seal member, but the liquid component does not leak to the outside because the space facing the surface of the seal member is sealed from the outside by the container adapter and the insertion portion of the needle cap.

In the two-component-mixing prefilled syringe kit according to the present invention, the tubular container adapter has a flange portion being in a direction intersecting with a barrel axis direction of the syringe barrel on an outer side surface of the seal member side, and the holding member has a mount portion for mounting the syringe barrel, and a pushing portion for pushing the flange portion against the container when the syringe barrel mounted on the mount portion is moved along the barrel axis direction to the container side, and a lock part for holding a state where the pushing portion pushes the flange portion against the container.

In the present invention, the holding member with the syringe barrel mounted on the mount portion is moved to the container side along the barrel axis direction, and the tip of the injection needle pierces the insertion portion on the front portion side of the needle cap and the seal member of the container outlet, and the flange portion of the outer side surface of the front portion of the needle cap that intersects the barrel axis direction of the syringe barrel is pushed against the container by the pushing portion provided on the holding member, the state where the pushing portion pushed the flange portion against the container is held by the lock part provided on the holding member. As a result, the tip of the injection needle pierces the insertion portion of the needle cap and the seal member of the container, and by the tubular container adapter fixed in close contact with the surface of the seal member and the insertion portion of the needle inserted so as to be in close contact with the inner surface of the container adapter, the state where the surface of the seal member faces the space sealed from the outside is stably maintained.

Further, when the syringe barrel containing the prepared injection solution is taken out to the outside along the barrel axis direction, as the flange portion of the needle cap is pushed against the container side and clamped by the pushing portion of the holding member, the needle cap fixed at a base end side to the base of the injection needle or the outer surface of the syringe barrel is forcibly separated from the syringe barrel and remains inside of the prefilled syringe kit, and the syringe with the needle cap removed can be taken out to the outside. As a result, it is not necessary to remove the needle cap when using the syringe.

A two-component-mixing prefilled syringe kit according to the present invention is characterized that; it comprises a needle cap which is formed of a material that can be pierced by the injection needle to cover the injection needle from the tip end side, and fixed at the base end side to the base of the injection needle or the outer surface of the syringe barrel; and a container adapter having a tubular portion which is fixed in close contact with the surface of the seal member at one end in the tube axis direction and a flat plate-shape main body portion which covers an opening on the other end side in the tube axis direction of the tubular portion; the main body portion of the container adapter is formed of a material that is pierceable by the injection needle at a portion facing the seal member; and the space-forming body is constituted by the container adapter.

In the present invention, when one end in the tube axis direction of the tubular portion of the container adapter is fixed in close contact with the surface of the seal member, since the opening on the other end side in the tube axis direction of the tubular portion is covered by the flat plate-like main body portion, an internal space of the tubular portion that the surface of the seal member faces is sealed from the outside. The container and the syringe barrel approach each other, the front portion of the needle cap comes in contact with the flat plate-like main body portion of the container adapter, and further, the tip of the injection needle pierces the expected piercing place at the front portion of the needle cap and the seal member at the outlet of the container pierces contacts the front part of the needle cap and a portion facing the seal member in the main body portion of the container adapter, and passing through the inside of the tubular portion of the container adapter to pierce the seal member at the container outlet, after the inside of the container and the inside of the syringe barrel are communicated, the liquid component in the container is moved to the inside of the syringe barrel to prepare the injection solution. When pulling out the injection needle pierced the seal member of the container from the seal member in order to separate the syringe barrel containing the prepared injection solution from the container, the liquid component remaining in the container may leaks to the surface of the seal member, but as the space which the surface of the seal member faces is sealed from the outside by the container adapter, the liquid component does not leak to the outside.

The two-component-mixing prefilled syringe kit according to the present invention is characterized that; the container adapter has a flange portion being in a direction intersecting with a barrel axis direction of the syringe barrel on an outer surface of the seal member side, and the needle cap has a flange portion being in a direction intersecting with a barrel axis direction of the syringe barrel on an outer side surface at the front side, and the holding member has a mount portion for mounting the syringe barrel and a pushing portion for pushing two flange portions of the needle cap and the container adapter against to the container in overlapped state when moving along the barrel axis direction of the syringe barrel to the container side, and a lock part for holding the state where the pushing portion pushes the two flange portions against to the container.

In the present invention, when the holding member with the syringe barrel mounted on the mount portion is moved toward the container along the barrel axis, the tip of the injection needle pierces the front portion of the needle cap, the main body portion of the container adapter and the seal member of the container, the two flange portions of the flange portion on the outside of the front portion the needle cap and the flange portion on the outer side surface of the container adapter are pushed against the container by the pushing portion provided on the holding member, and a state where the pushing portion pushes the two flange portions against the container is held by the lock part provided on the holding member. As a result, the tip of the injection needle pierces the front portion of the needle cap, the main body portion of the container adapter and the seal member of the container, and a state where the surface of the seal member facing the space sealed from the outside is stably maintained by the container adapter fixed in close contact with the surface of the seal member.

Furthermore, when taking out the syringe barrel containing the prepared injection solution to the outside along the barrel axis direction, as the flange portion of the needle cap and the flange portion of the container adapter are pushed against the container side and clamped by the pushing portion of the holding member, the needle cap fixed at base end side to the base side of the injection needle or the outer surface of the syringe barrel is forcibly separated from the syringe barrel and remains inside the prefilled syringe kit, the syringe with the needle cap removed can be taken out. As a result, it is not necessary to remove the needle cap when using the syringe.

The length of the tubular portion along the tubular axis direction is less than 4 mm.

Because, if it is 4 mm or more, the moving distance required for the needle to pass from the needle cap through the inside of the tubular portion of the container adapter and pierces the seal member of the container becomes long, and the operability is deteriorated.

A two-component-mixing prefilled syringe kit according to the present invention is characterized that the lock part is a claw body that can be engaged with a concave portion or a step portion on the outer surface of the container.

In the present invention, since the claw body that is the lock part engages with the concave portion or the step portion of the outer surface of the container, a state that the pushing portion of the holding member pushes the flange portion of the needle cap against the container is maintained by a simple mechanical mechanism.

A two-component-mixing prefilled syringe kit according to the present invention is characterized by comprising an accommodating case that accommodates the whole of the syringe barrel, the needle cap, the container and the holding member in a sealed state to the outside.

Alternatively, a two-component-mixing prefilled syringe kit according to the present invention is characterized by comprising an accommodating case that accommodates the whole of the syringe barrel, the needle cap, the container adapter, the container and the holding member in a sealed state to the outside.

In the present invention, the accommodating case can more effectively prevent the leakage to the outside of the constituents of the injection solution filled in the container and the syringe barrel, including the leakage of the residual liquid in the container.

A two-component-mixing prefilled syringe kit according to the present invention is characterized that the tip of the injection needle is held in the needle cap in a non-penetrated piercing state.

In the present invention, since the tip of the injection needle is held in the needle cap in a non-penetrating state, the tip of the injection needle is located inside the material of the needle cap and is not exposed to the outside. It is possible to store the injection needle and the syringe barrel attached with the injection needle in a stable posture while avoiding the outflow to the outside of the constituent components of the injection solution filled in the syringe barrel.

### Effect of the invention

According to the two-component-mixing prefilled syringe kit according to the present invention, when an injection solution is prepared by mixing the drug component in the syringe barrel and the liquid component in the container, it is possible to effectively prevent the leakage to the outside of the liquid component remaining in the container after the injection solution is prepared.

### Brief description of the drawings

[Figure 1] a vertical section of a two-component-mixing prefilled syringe kit according to an embodiment of the present invention.
[Figure 2] a vertical section of a two-component-mixing prefilled syringe kit according to an embodiment of the present invention.
[Figure 3] a perspective drawing which shows an external appearance and an internal structure of a needle cap.
[Figure 4] a perspective drawing which shows structure of a holding member.
[Figure 5] an inner surface side perspective drawing of an accommodating case and a dimension explanatory diagram of operating ribs.
[Figure 6] a vertical section, a partially enlarged section and a partially assemble drawing of members for making an accommodating case.
[Figure 7] a sectional arrow-view drawing at VII-VII position of Figure 1.
[Figure 8] a sectional arrow-view drawing which shows the position of the accommodating case that rotates against the syringe barrel.
[Figure 9] a perspective drawing which shows an assembly process of the syringe with the holding member.
[Figure 10] a perspective drawing showing an assembly process of the prefilled syringe kit shown in Figure 1.
[Figure 11] a section explaining the preparation operation of the injection solution by the prefilled syringe kit of Figure 1.
[Figure 12] a partially enlarged vertical section of Figure 11(c) and a dimension explanatory drawing of the tubular portion at the tip of the needle cap.
[Figure 13] a perspective drawing showing a process for preparing an injection solution by the prefilled syringe kit of Figure 1.
[Figure 14] a perspective drawing showing a process for preparing an injection solution by the prefilled syringe kit of Figure 1.
[Figure 15] a perspective drawing showing take-out of syringe from prefilled syringe kit of Figure 1.
[Figure 16] a perspective drawing showing a disposal preparation process of a used syringe.
[Figure 17] a schematic section enlarging a part of the accommodating case after the disposal preparation process is completed.
[Figure 18] a partially enlarged section of the prefilled syringe kit according to a 1st another embodiment of present invention.
[Figure 19] a perspective drawing showing an assembly process of the syringe with the holding member.
[Figure 20] a perspective drawing showing an assembly process of the prefilled syringe kit shown in Figure 18.
[Figure 21] a section explaining the injection solution preparation operation by the prefilled syringe kit of Figure 18.
[Figure 22] a partially enlarged section of the prefilled syringe kit according to a 2nd another embodiment of present invention.
[Figure 23] a section explaining the injection solution preparation operation by the prefilled syringe kit of Figure 22.
[Figure 24] a partially enlarged vertical section of a prefilled syringe kit according to a third another embodiment of the present invention.
[Figure 25] a perspective drawing showing an assembly process of the prefilled syringe kit shown in Figure 24.

### Mode for carrying out the invention

Hereinafter, embodiments of a two-component-mixing prefilled syringe kit according to the present invention will be described with reference to the drawings.

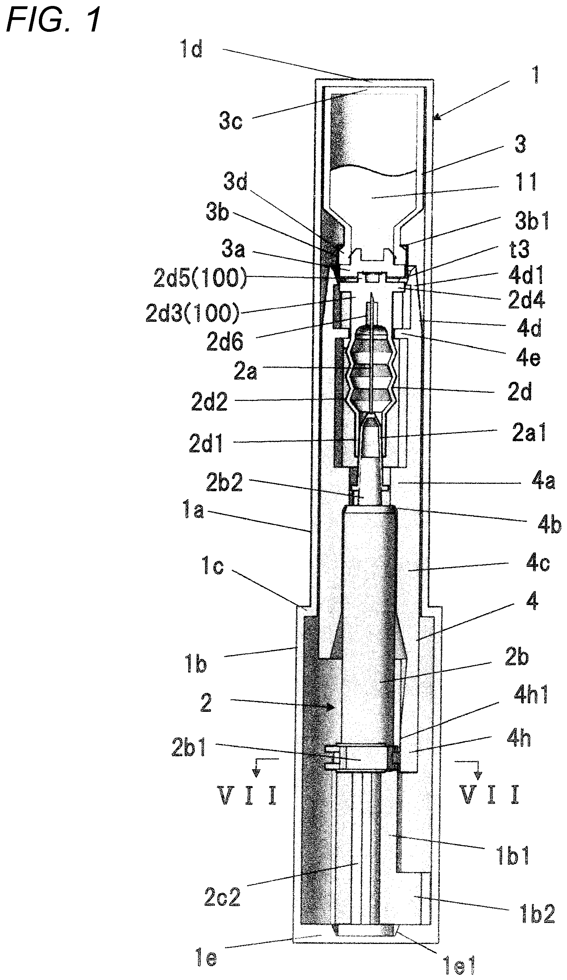

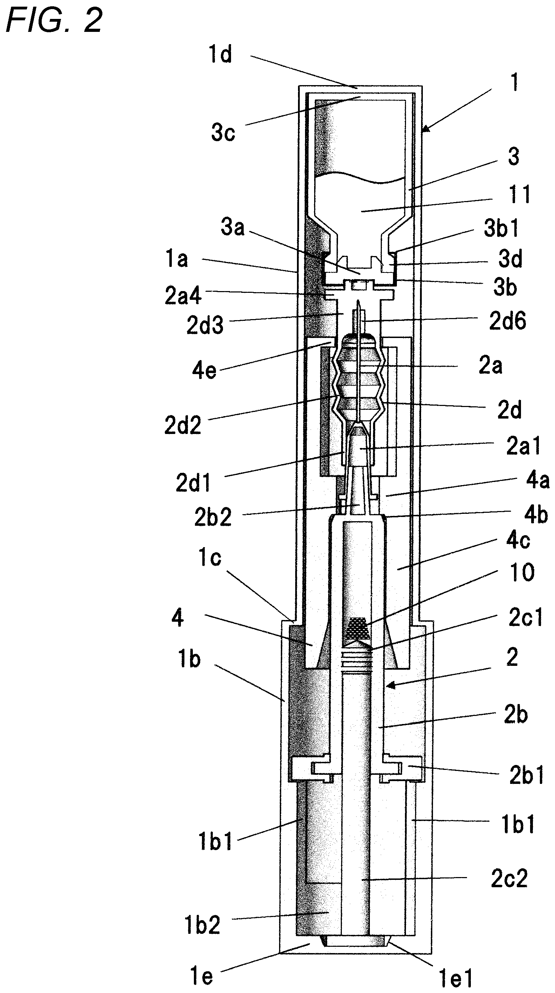

Fig.1 and Fig.2 are vertical sections of a two-component-mixing prefilled syringe kit according to the present invention. Also, Fig. 1 and Fig.2 show vertical sections at positions where they are rotationally symmetrical by 90 degrees to each other in a cross-section of the prefilled syringe kit.

a prefilled syringe kit shown in Fig.1 and Fig.2 includes a cylindrical accommodating case 1 which is elongated in an axial direction. The accommodating case 1 has a structure that in a state where a small-diameter cylindrical portion 1a having a small outer diameter and a large-diameter cylindrical portion 1b having a large outer diameter are aligned with their axes each other, the outer circumference of one end of the small-diameter cylindrical portion 1a and the inner circumference of one end of the large-diameter cylindrical portion 1b are connected by an toroidal thin plate portion 1c. The other ends of the cylindrical portions 1a and 1b are respectively closed by walls 1d and 1e. As a result, the inside of the accommodating case 1 is sealed from the outside. The accommodating case 1 is made of a transparent hard vinyl chloride resin or the like.

Inside the accommodating case 1, the syringe 2, the holding member 4 and the container 3 are sequentially arranged along the axial direction of the accommodating case 1. The holding member 4 holds the syringe 2 to position the syringe 2 with respect to the container 3. Inside the small-diameter cylindrical portion 1a, the container 3, most of the holding member 4 and the front-side part of the syringe 2 are located, and inside the large-diameter cylindrical portion 1b, a part of the holding member 4 and the rear-side part of the syringe 2 are located.

The syringe 2 comprises a syringe barrel 2b, an injection needle 2a attached to a front end portion 2b2 of the syringe barrel 2b, a plunger (also referred to as a gasket) 2c1 inserted into a rear end opening of the syringe barrel 2b, and the driving rod 2c2 connected a rear portion of the plunger 2c1. The injection needle 2a is fixed with the flange portion 2a1 of the base side pushed into the tip end side of the front end portion 2b2 of the syringe barrel 2b. The syringe barrel 2b is made of glass such as borosilicate glass. The plunger 2c1 is generally made of butyl rubber, and is coated with silicone oil in order to smoothly slide on the inner surface of the syringe barrel 2b. As a result, the inner surface of the syringe barrel 2b that contacts the plunger 2c1 is also coated with silicone oil. Also, the silicone oil coating method is generally a method that a gasket is put into water dispersing silicone oil and stirred.

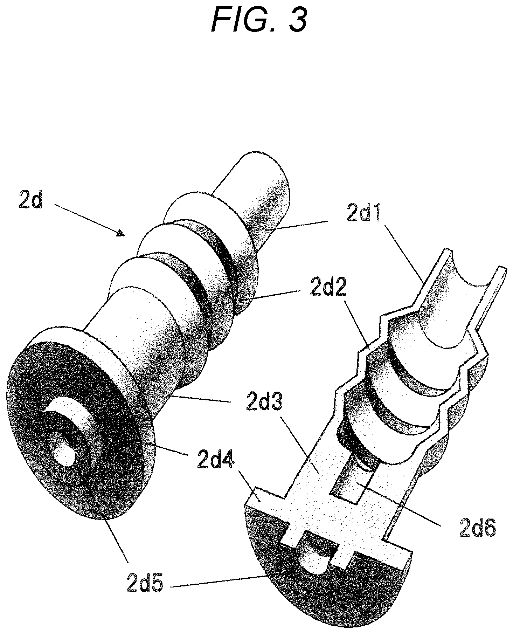

A needle cap 2d which cover the injection needle 2a from the tip side is provided. Fig.3 is a perspective drawing showing the appearance and internal structure of the needle cap. The needle cap 2d has a structure that a cylindrical portion 2d1 on the base end side, a bellows portion 2d2 having an outer diameter larger than that of the cylindrical portion 2d1, and a cylindrical front portion 2d3 having an outer diameter intermediate between the outer diameter of the cylindrical portion 2d1 and the outer diameter of the bellows portion 2d2 continue sequentially coaxially along the axial direction of the cylindrical portion 2d1. The front portion 2d3 is provided with a disc-shaped flange portion 2d4 on the side surface of the tip end portion, and is provided with a cylindrical tubular portion 2d5 having a diameter smaller than the outer diameter of the front portion 2d3 on the front surface of the tip end portion. The tip of the tubular portion 2d5 is located on one plane orthogonal to the axial direction of the syringe barrel 2b. Further, a cylindrical cavity 2d6 is formed in the front portion 2d3 from an end on the side of the bellows portion 2d2 to an intermediate position in the axial direction, and the cavity 2d6 communicates with the inner space of the cylindrical portion 2d1 and the bellows portion 2d2. The needle cap 2d is made of butyl rubber, for example. The tip of the injection needle 2a is pierced through the cavity 2d6 into the tip side portion of the front portion 2d3 in a non-penetrating state. Also, the open end of the cylindrical portion 2d1 is fitted and held on the outer periphery of the flange portion 2a1 of the injection needle 2a.

The container 3 is a cylindrical container having an outer diameter slightly smaller than the inner diameter of the small-diameter cylindrical portion 1a of the accommodating case 1, and is made of glass such as borosilicate glass. The container 3 is specifically a vial. The outlet of the container 3 is sealed by a seal member 3a. The seal member 3a is fixed by a stopper 3b so as to close the outlet opening of the container 3. The stopper 3b includes a circular plate portion having a central part opening, and a cylindrical base portion that is continuous with the peripheral edge of the circular plate portion and extends toward the bottom side of the container 3. The circular plate portion of the stopper 3b pushes the seal member 3a against the outlet of the container 3. The cylindrical base portion of the stopper 3b is narrowed inward so that the tip side surrounds the flange portion 3d on the outlet side of the container 3 to form a step portion 3b1. The seal member 3a is made of butyl rubber or chlorinated butyl rubber, and the stopper 3b is made of metal or hard plastic.

a powder drug 10 is filled in the inside of the syringe barrel 2b as a first component constituting the injection solution. The powder drug is crystalline powder, freeze-dried powder, or the like. On the other hand, in the inside of the container 3, a solution 11 for dissolving the powder drug 10 or a dispersion liquid 11 for dispersing the powder drug 10 is filled as a second component constituting the injection solution. In addition, both the first component and the second component may include multiple types of substances other than one type of substance.

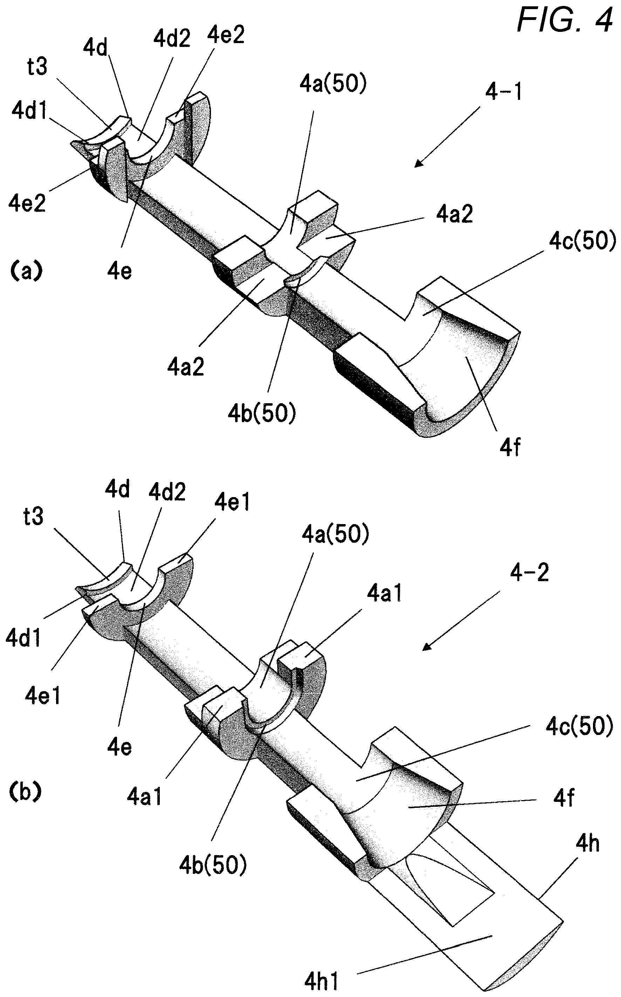

Next, the holding member 4 is described. Fig.4 is a perspective drawing showing the structure of the holding member 4.

The holding member 4 is a cylindrical member having an outer diameter slightly smaller than the inner diameter of the small-diameter cylindrical portion 1a of the accommodating case 1, and is constituted by two semi-cylindrical members 4-1, 4-2 which are vertically divided by a plane passing through the axis (Fig. 4(a) (b)). The syringe 2 is housed inside the holding member 4 with the barrel axes aligned. The holding member 4 is made of a transparent hard vinyl chloride resin or the like.

The holding member 4 is provided with a toroidal rib 4a which holds the flange-shaped toroidal part at the rear end of the flange portion 2a1 of the base side of the injection needle 2a, and a toroidal step portion 4b which continues to the rear side of the toroidal rib 4a, being in contact with the peripheral corner portion to the front side of the syringe barrel 2b to regulate the position, and a cylindrical guide 4c which has an inner diameter slightly larger than the outer diameter of the syringe barrel 2b, to regulate the position in the radial direction of the syringe barrel 2b on the rear side of the toroidal step portion 4b. That is, the toroidal rib 4a, the toroidal step portion 4b, and the cylindrical guide 4c constitute the mount portion 50 provided in the holding member 4. At the rear end portion of the holding member 4, a cylindrical guide 4f having a diameter increased toward the rear end side is provided to guide the insertion of the used syringe 2. Further, on one of the semi-cylindrical members 4-2 (Fig. 4(b)), a rotation stop portion 4h having a crescent section is extended from the end of the cylindrical guide 4f in the barrel axial direction. The rotation stop portion 4h has a flat-face inner peripheral surface 4h1 parallel to the barrel axis direction.

A movable piece 4d is provided at the front end of the holding member 4, and a toroidal plate-shaped pushing portion 4e protruding inward is provided at a base side place of the movable piece 4d. The movable piece 4d includes a hook portion 4d1 at the tip and a support portion 4d2 which supports the hook portion 4d1 and is elastically bendable in the radial direction. The movable pieces 4d are provided at two locations of 180-degree rotational symmetric position in a cross section orthogonal to the barrel axis. The hook portion 4d1 is formed with an inclined surface t3 that is inclined so as to form an acute angle with respect to the barrel axis center direction and has a surface direction facing the barrel axis center side.

In one semi-cylindrical member 4-1, the pushing portion 4e is formed by a half circumference, and a pair of step portions 4e2, 4e2 are formed at 180-degree rotational symmetry positions by parts which are respectively axially adjacent and radially protrude from both circumferential edges of the pushing portion 4e. In the other semi-cylindrical member 4-2, the pushing portion 4e is formed by a half circumference, and a pair of convex portions 4e1, 4e1 protruding in the circumferential direction are formed at both edges. Further, in the one semi-cylindrical member 4-1, an axial rear portion of the toroidal rib 4a is formed shorter than a half circumference, and a pair of step portions 4a2, 4a2 having low height are formed. In the other semi-cylindrical member 4-2, the rear half in the axial direction of the toroidal rib 4a is formed longer than the half circumference, and a pair of step portions 4a1, 4a1 having high height are formed. Then, the semi-cylindrical member 4-1 shown in Fig.4(a) is turned upside down (turned over) and overlapped on the semi-cylindrical member 4-2 shown in Fig. 4(b), inset each of the pair of convex portions 4e1, 4e1 into each of the pair of the step portions 4e2, 4e2 at the location of the pushing portion 4e, and strike to fit together each of the pair of step portions 4a1, 4a1 into each of the pair of the step portions 4a2, 4a2 at the location of the toroidal rib 4a, so that both of the semi-cylindrical members 4-1, 4-2 are joined together to form a cylindrical holding member 4.

As shown in Fig.1 and Fig.2, the bottom portion 3c on the side opposite to the seal member 3a of the container 3 is in contact with the wall 1d of the accommodating case 1, so that the axial position of the container 3 is regulated. Also, the inclined surface t3 of the hook portion 4d1 at the tip of the movable piece 4d of the holding member 4 is in contact with the peripheral corner portion of the stopper 3b of the container 3. As a result, the syringe 2 is positioned with respect to the container 3 in a state where the tip of the injection needle 2a pierced the needle cap 2d in a non-penetrate state faces the seal member 3a of the container 3 via the needle cap 2d.

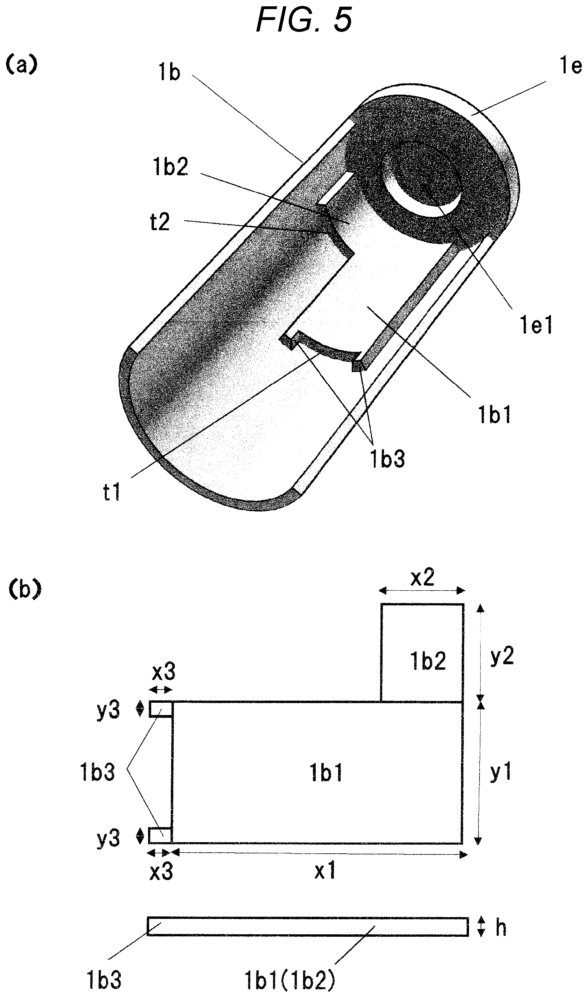

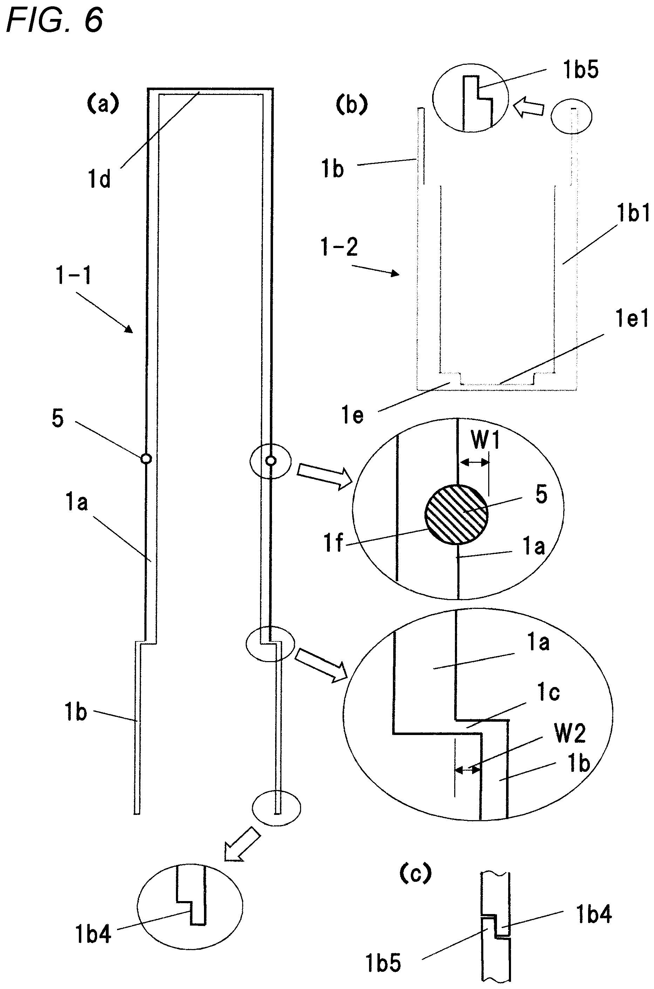

A structure of the accommodating case 1 is described by Fig.5 and Fig,6. Fig.5 is a perspective drawing of the inner-surface side of the accommodating case and an explanatory drawing of dimensions of the operating ribs, and Fig.6 is a vertical section and a partially enlarged section and a partially assemble drawing of members for manufacturing the accommodating case. Fig.5(a) shows the inner surface side of the accommodating case 1 in which the large-diameter cylindrical portion 1b is virtually split vertically along the axial direction, and a half of the barrel is cut away except for the wall 1e. Fig.5(b) shows the dimensions of the ribs developed in the circumferential direction.

A rib 1b1 and a second rib 1b2 protruding from the inner circumferential wall at a uniform height h are provided on the inner surface side of the large-diameter cylindrical portion 1b. The rib 1b1 is a stepped portion having a length x1 from the wall 1e and a height h protruding in a rectangular shape with a circumferential width y1. The second rib 1b2 is adjacent to the rib 1b1 in the circumferential direction, and is a step of h which has a length from the wall 1e that is x2 shorter than the length x1 of the rib 1b1 (x1>x2), and protruding in a rectangular shape with a circumferential width y2. The rib 1b1 has a side surface t1 perpendicular to the barrel axis direction on the side opposite to the wall 1e. The second rib 1b2 has a side surface t2 perpendicular to the barrel axis direction on the side opposite to the wall 1e. A pair of third ribs 1b3 having a length x3 in the axial direction, a width y3 in the circumferential direction, and a height h is provided at both ends in the circumferential direction of the side surface t1 of the rib 1b1. The third rib 1b3 is elongated in the barrel axis direction, and the length x3 in the barrel axis direction is set to be approximately the same as the thickness of the flange portion 2b1 at the rear end of the syringe barrel 2b. The ribs 1b1, the second ribs 1b2, and the third ribs 1b3 are provided in pairs at positions 180 degrees rotationally symmetrical in the horizontal cross section of the accommodating case 1, respectively.

FIG. 6(a) shows a case component 1-1 having a small-diameter cylindrical portion 1a and a part of the large-diameter cylindrical portion 1b connected to the small-diameter cylindrical portion 1a by a thin plate portion 1c. In the case component 1-1, the end on the side of the large-diameter cylindrical portion 1b is opened, and the end on the side of the small-diameter cylindrical portion 1a becomes the wall 1d. FIG. 6(b) shows a case component 1-2 made up of the remaining part of the large-diameter cylindrical portion 1b. In the case component 1-2, one end in the axial direction is open and the other end becomes the wall 1e.

A concave groove 1f having a semicircular cross section is formed on the outer circumferential location of the small-diameter cylindrical portion 1a over the entire of circumferential direction thereof, and an O-ring 5 is fitted into the concave groove 1f. The protrusion amount W1 of the O-ring 5 from the outer circumferential surface of the small-diameter cylindrical portion 1a is arranged smaller other than the difference of positions in the radial direction between the outer circumferential surface of the small-diameter cylindrical portion 1a and the inner circumferential surface of the large-diameter cylindrical portion 1b (that is, a width of the toroidal thin plate portion 1c) W2 (W1>W2).

A step portion 1b4 having a shape in which a thick outer portion of the large-diameter cylindrical portion 1b protrudes in the axial direction is formed over the entire circumference at an opening-side edge of the case component 1-1. On the other hand, a step portion 1b5 having a shape in which a thick inner portion of the large-diameter cylindrical portion 1b protrudes in the axial direction is formed over the entire circumference at an opening-side edge of the case component 1-2.

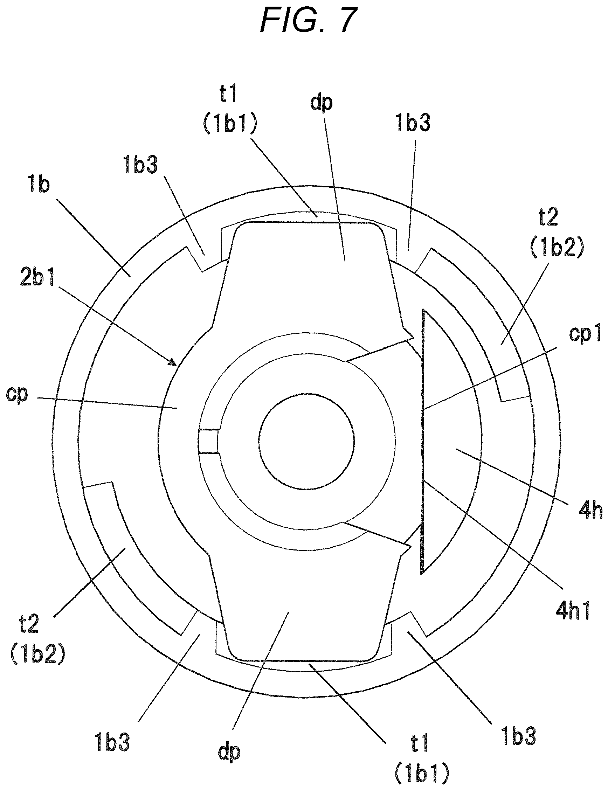

Next, based on FIGS. 1 and 7, the positioning and holding structure of the rear side of the syringe 2 by the accommodating case 1 will be described. FIG. 7 is a cross-sectional arrow drawing at the position VII-VII in FIG. 1

The flange portion 2b1 at the rear end of the syringe barrel 2b has an outer shape in which trapezoidal portions dp narrowing outward are connected to both sides that are rotationally symmetrical by 180 degrees of the circular portion cp in the axial direction view. The circular portion cp has a straight outer circumferential portion cp1 on one end side in the direction orthogonal to the direction connecting the two trapezoidal portions dp. The edge portion of each trapezoidal portion dp of the flange portion 2b1 is in contact with the side surface t1 of the rib 1b1 between the pair of third ribs 1b3. As a result, the syringe barrel 2b is positioned in the axial direction, and when the syringe barrel 2b rotates around the axis center, it is regulated that each of the trapezoidal portions dp of the flange portion 2b1 does not come outside further other than the third ribs 1b3 by the third ribs 1b3 on both sides, and the flange portion 2b1 is held in a state of being in contact with the side surface t1 of the rib 1b1. Also, the inner circumferential surface 4h1 of the rotation stop portion 4h of the holding member 4 is in contact with the straight outer circumferential portion cp1 of the flange portion 2b1, whereby the syringe barrel 2b and the holding member 4 rotate integrally around the axis center.

A circular concave portion 1e1 having a constant depth is formed in the center of the inner surface of the wall 1e. The diameter of the concave portion 1e1 is larger than the outer shape of the flange at the rear end of the driving rod 2c2, and the depth of the concave portion 1e1 is approximately the same as the thickness of the flange at the rear end of the driving rod 2c2. The flange at the rear end of the driving rod 2c2 is in contact with the bottom of the concave portion 1e1 and the axial position of the driving rod 2c2 and thus the plunger 2c1 is restricted.

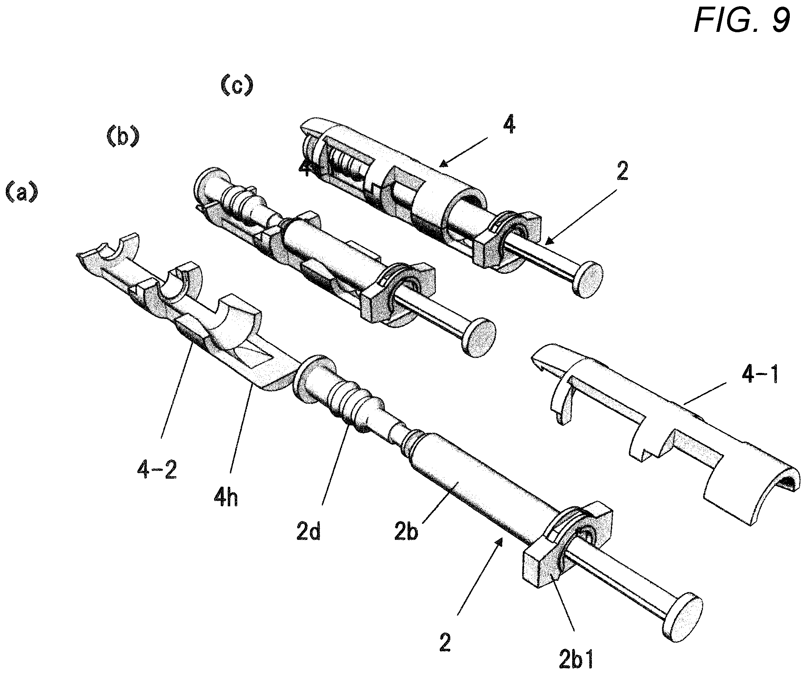

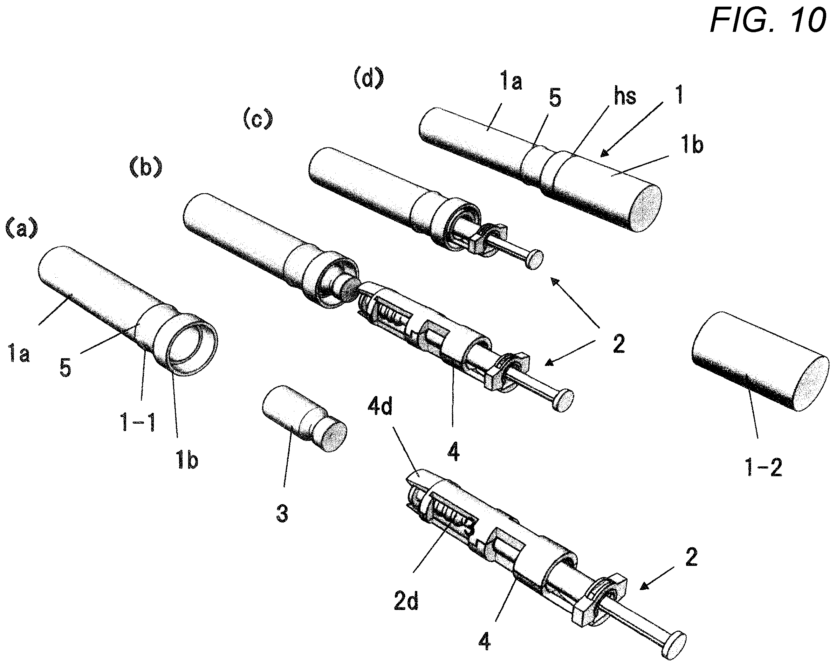

Next, the assembly of the prefilled syringe kit will be described. FIG. 9 is a perspective drawing showing an assembling process of the syringe to the holding member, and FIG. 10 is a perspective drawing showing an assembling process of the prefilled syringe kit.

In the step of assembling the syringe 2 to the holding member 4, first, the syringe 2 prefilled with the powder drug 10 and having the needle cap 2d attached thereto is set from above to the semi-cylindrical member 4-2 with the inside facing upward. (FIG (a) and (b)). At this time, the position is set so that the straight outer circumferential portion cp1 of the flange portion 2b1 comes into contact with the rotation stop portion 4h of the holding member 4 (see FIG. 7). Next, the semi-cylindrical member 4-1 with the inside facing downward is put over the syringe 2 and integrally coupled with the semi-cylindrical member 4-2 (FIGS.(b)and(c). As a result, the holding member 4 is formed by the semi-cylindrical members 4-1 and 4-2 that are integrally connected, and the syringe 2 is assembled to the holding member 4 with the syringe barrel 2b fixed and supported by the holding member 4.

In the assembling process of the prefilled syringe kit, first, the container 3 is inserted from the bottom side into the end opening on the large-diameter cylindrical portion 1b side of the case component 1-1 (FIG. 10(a)). Subsequently, the holding member 4 on which the syringe 2 with the needle cap 2d is set is inserted to the inside of the case component 1-1 with the movable piece 4d facing forward (FIGS. 10(a)(b)(c)). Next, the case component 1-2 is covered so as to cover the rear side of the syringe 2 that has popped out of the end opening of the case component 1-1, and the opening ends of both case components 1-1 and 1-2 are joined so that the both step portions 1b4 and 1b5 are fitted into each other (FIGS.10(c)(d) and FIG. 6(c). At this time, the cylindrical tubular portion 2d5 of the tip of the needle cap 2d is became into a state of being close to and facing the seal member 3a of the container 3. Further, as shown in FIG. 7, with the flange portion 2b1 of the syringe barrel 2b in contact with the rib 1b1, the two trapezoidal portions dp on both sides are held between the pair of third ribs 1b3 to regulate the rotational position.

Finally, the step portions 1b4 and 1b5 of both the joined case components 1-1 and 1-2 are thermally joined to form a joint portion hs (FIG. 10(d)), and the sealed case 1 is configured. The thermal bonding is, for example, an electric iron method, in this method the accommodating case 1 is rotated with respect to the fixed-type iron while pressing the iron heated to high temperature on the joint part of the case components 1-1 and 1-2 (or by rotating the iron with respect to the fixed-type accommodating case 1), the material (vinyl chloride resin) of the accommodating case 1 is welded. Further, it is possible to use a method in which high temperature hot air is blown onto the joint part of the case components 1-1 and 1-2 to weld them together.

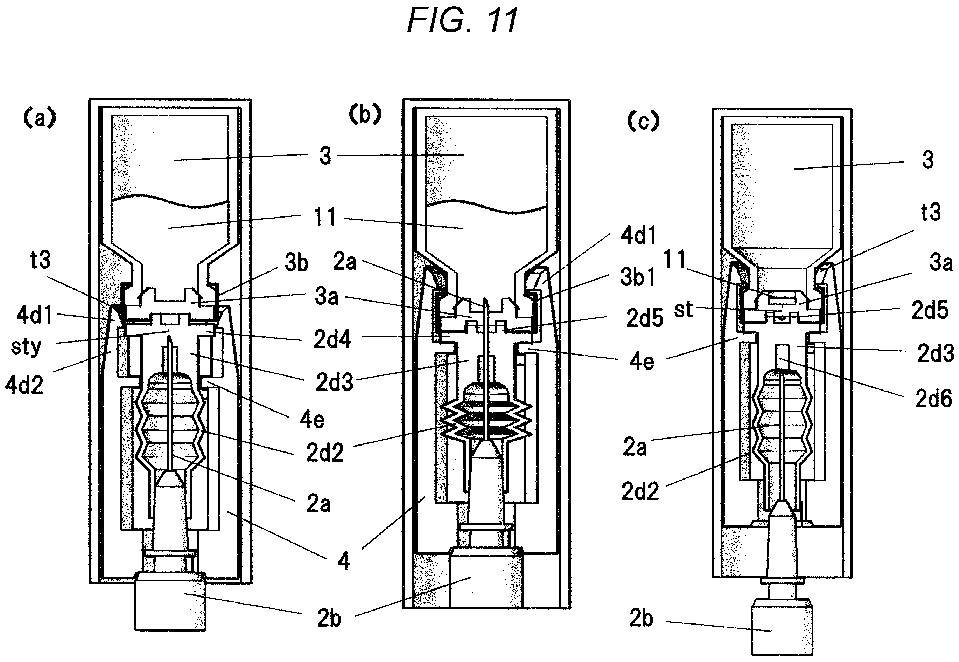

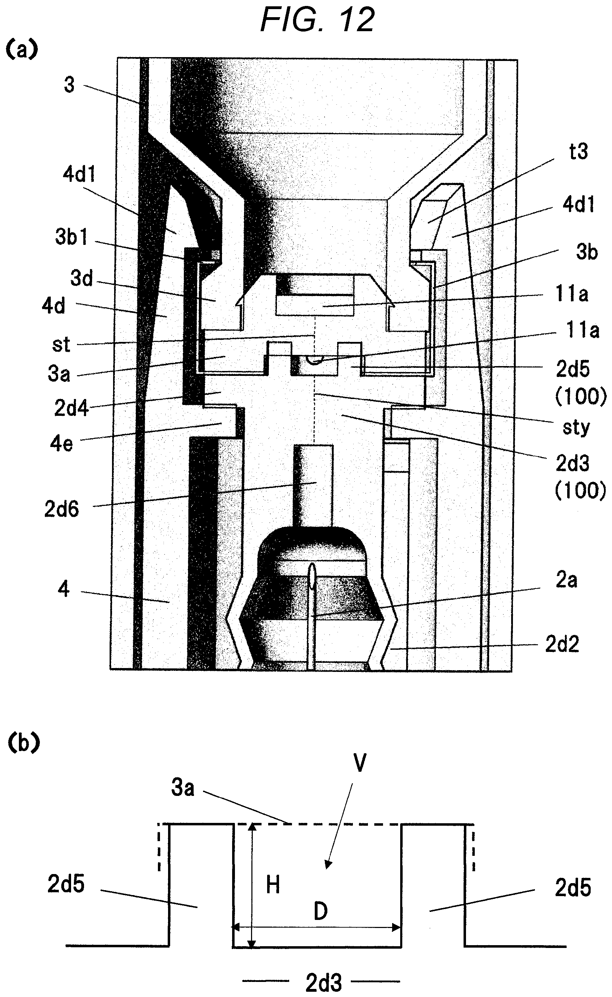

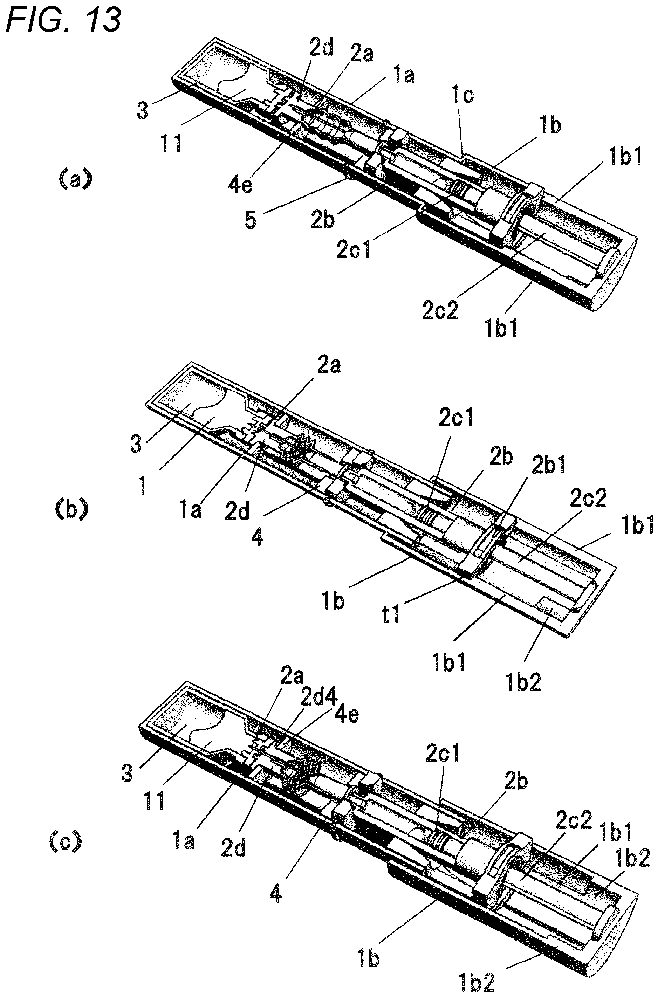

Next, an operation for preparing an injection solution by the two-component-mixing prefilled syringe kit according to the present invention is described. FIG. 11 is a cross-sectional drawing for explaining the injection solution preparation operation by the prefilled syringe kit of FIG. 1, and FIG. 12 is a partially enlarged cross-sectional drawing of FIG. 11(c) and a dimension explanatory drawing of the tubular portion at the tip of the needle cap. Note that in FIGS. 11 to 12 (the same applies to FIGS. 13 to 15, FIG. 18, and FIGS. 21 to 23), for convenience of description, the accommodating case 1 is virtually vertical divided along the axial direction and a half of the cylindrical portion is removed, and it is drawing so that the inside is visible.

When the syringe barrel 2b is pushed into the container 3 side, the bellows portion 2d2 of the needle cap 2d contracts, and the tip of the injection needle 2a that has been pierced to the midway point of the front portion 2d3 of the needle cap 2d penetrates the needle piercing point Sty and the seal member 3a and projects inside the container 3, the tubular portion 2d5 of the front portion 2d3 of the needle cap 2d is pushed against the seal member 3a (FIGS. 11(a) and(b)). At this time, the holding member 4 is also pushed by the syringe barrel 2b and moved to the container 3 side, and the hook portion 4d1 of the movable piece 4d which is in contact with the peripheral corner portion of the stopper 3b of the container 3 on the inclined surface t3, slides to move outward, and the supporting portion 4d2 bends outward, and the hook portion 4d1 that has moved outward passes by sliding on the side surface of the stopper 3b. At the same time, the pushing portion 4e of the holding member 4 pushes the flange portion 2d4 of the needle cap 2d against the front surface of the stopper 3b of the container 3. Then, the hook portion 4d1 that has slid outwardly returns to the inside and is hanged by the step portion 3b1 on the base side of the stopper 3b, and the holding member 4 is mechanically coupled to the container 3 at this position. As a result, the state in which the tip of the injection needle 2a projects into the container 3 and the state in which the tubular portion 2d5 of the front portion 2d3 of the needle cap 2d is pushed against the seal member 3a are maintained (locked). That is, the hook portion 4d1 provided on the movable piece 4d corresponds to the lock portion that locks the state in which the pushing portion 4e is in contact with the container 3 and the claw body that can be engaged with the step portion 3b1 on the outer surface of the container 3.

Next, the driving rod 2c2 is operated with a finger to push the plunger 2c1, after the air in the syringe barrel 2b is sent into the container 3 to increase the pressure in the container 3, the finger is released from the driving rod 2c2 and the plunger 2c1 is made free, then the liquid 11 in the container 3 is sucked to inside of the syringe barrel 2b through the injection needle 2a by the internal pressure, and the plunger 2c1 is returned to the original position. The liquid 11 sucked into the syringe barrel 2b is mixed-type with the powder drug 10 inside the syringe barrel 2b to prepare the injection solution. Finally, the syringe barrel 2b is gripped with finger, the injection needle 2a is pulled out from the container 3, and is pulled back to the inside of the needle cap 2d (FIG. 11(c), FIG. 12(a)). At this time, a part of the liquid 11a of the liquid 11 remaining in the container 3 may overflow onto the surface of the seal material 3a through the needle piercing point St of the seal material 3a, but, as the space surrounded by the front portion 2d3 pushed against the seal material 3a of the needle cap 2d and the tubular portion 2d5 and the seal member 3a is sealed from the outside, the liquid 11a overflowing from the container 3 does not leak to the outside. That is, the space-forming body 100 is configured by the front portion 2d3 of the needle cap 2d and the tubular portion 2d5.

Next, a space V (closed space) surrounded by the surface of the seal member 3a and the front portion 2d3 and the cylindrical portion 2d5 of the needle cap 2d is described by FIG.12(b). Here, it is assumed that the tubular portion 2d5 is cylindrical. D is the inner diameter of the tubular portion 2d5, and H is the height (length along the tubular axis direction) of the tubular portion 2d5. The tubular portion 2d5 pushed against the seal member 3a is compressed in the pushing direction and is slightly deformed, but the effect of this deformation is not taken into consideration in the description.

Generally, the seal member 3a of the vial 3 has a circular shape in a plane view, and the size (diameter) of the area assigned to the needle piercing part of the seal member 3a is about 7 mm. When the tubular portion 2d5 is pushed into the area of the needle piercing part, assuming the thickness of the circumferential wall of the tubular portion 2d5 is 1 mm, the inner diameter D of the tubular portion 2d5 is about 4 mm at maximum. The height H of the tubular portion 2d5 cannot be increased so much in consideration of downsizing of the device and manufacturing. For example, when the height H is 1 mm, the volume of the space V is 0.0125 ml when the inner diameter D is 4 mm, and the volume of the space V is 0.00314 ml when the inner diameter D is 2 mm. When the height H is increased to 3 mm and the inner diameter D is decreased to 3 mm, the volume of the space V becomes 0.0212 ml. The volume of the space V is calculated by the formula V= π (D/2)²H.

After filling 2 ml of purified water (hereinafter referred to as a liquid) in a vial 3 having an inner space of 4 ml, the vial 3 is turned upside down so that the seal member 3a is located on the lower side, and when the syringe 2 having the needle tip 27G is pierced to the seal member and 2 ml of air is injected into the vial 3, a liquid leakage from the seal member 3a is confirmed. Therefore, in a state that the tubular portion 2d5 at the tip of the needle cap 2d is pushed against the surface of the seal member 3a and set, the syringe 2 with the needle tip 27G is pierced into the front portion 2d3 of the needle cap 2d and is pierced seal member 3a passed through the inside of the tubular portion 2d5, and injecting 2 ml of air in a vial 3 in which 2 ml of liquid is filled. Table 1 below shows the test result of liquid leakage to the outside, needle movement distance (operability), compactness, and comprehensive evaluation. As an experimental condition, the inner diameter D was changed to 1.0 and 2.0 mm for the height H=1.0 mm of the tubular portion 2d5, and the inner diameter D was changed to 2.0 and 3.0 mm for the height H=2.0 mm of the tubular portion 2d5. Is changed to 2.0 and 3.0 mm, and the inner diameter D is 1.0, 2.0, 3.0 and 4.0 for each of the height H of the tubular portion 2d5 of 3.0 mm and 4.0 mm.

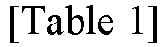

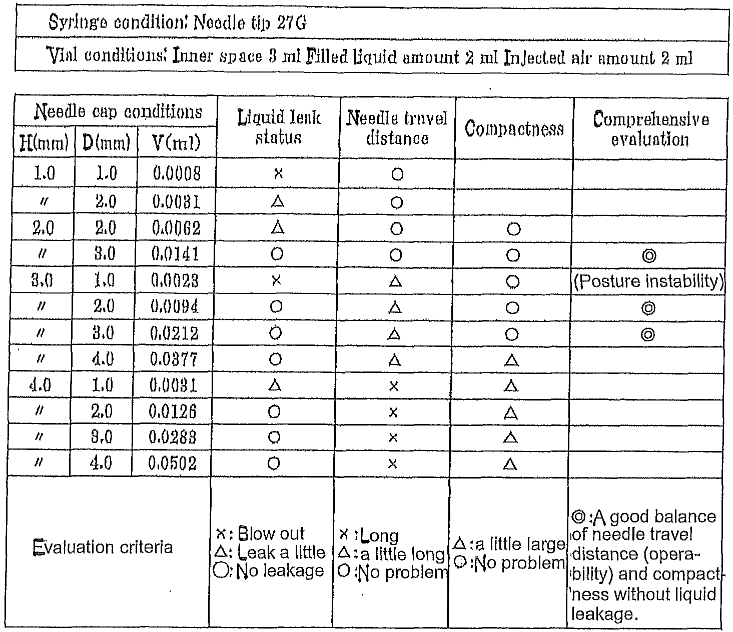

Regarding liquid leakage, there is no liquid leakage when the volume of the space V is larger than 0.0094 ml. The needle movement distance is a distance required to pierce the vial from the needle cap with the needle. Since the operability deteriorates when the height H of the tubular portion 2d5 becomes 4 mm or more, the allowable range is less than 4 mm. Compactness decreases as the size of the tubular portion 2d5 increases. Evaluating comprehensively that the needle movement distance (operability) and compactness are met well-balanced without liquid leakage, three conditions of an inner diameter D2.0 at height H3.0 mm and an inner diameter D2.0 at height H3.0 and an inner diameter D3.0 mm at height H3.0 of the tubular portion 2d5 were set as preferable conditions. Also, if the ratio of the height H to the inner diameter D is large, the posture when the tubular portion 2d5 is pushed against the surface of the seal member 3a may become unstable (for example, when the inner diameter D 1.0 mm at the height H3.0mm of the tubular portion 2d5). The volume of the space V is allowable in the range of 0.001 ml to 0.03 ml, and more preferably in the range of 0.005 ml to 0.025 ml. The inner diameter D is allowable in the range of 1.0 to 5.0 mm, and the more preferable range is 2.0 mm to 4.0 mm.

The tubular portion 2d5 is not limited to the cylindrical shape, and may be an elliptic tubular shape, a rectangular tubular shape, or the like.

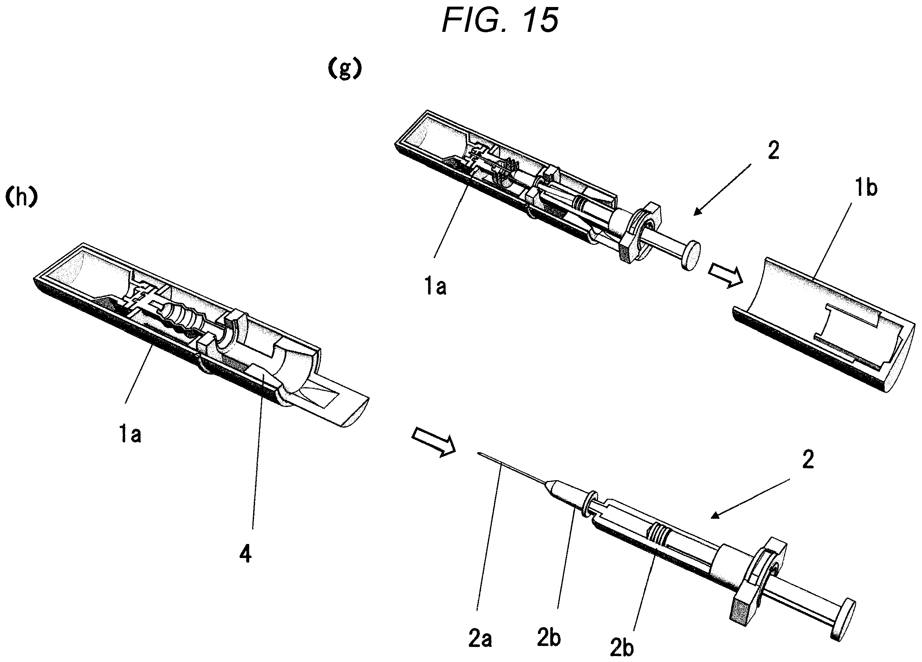

Next, the use of the two-component-mixing prefilled syringe kit according to the present invention is described in details. FIG. 13 to 14 are a perspective drawings showing an injection solution preparing process by the prefilled syringe kit of FIG. 1, and FIG. 15 is a perspective drawing showing taking out of the syringe from the prefilled syringe kit of FIG 1.

First, in the initial state (FIG. 13(a)), when the large-diameter cylindrical portion 1b of the accommodating case 1 is pushed into the small-diameter cylindrical portion 1a along the axial direction, the toroidal thin plate portion 1c is broken and the large-diameter cylindrical portion 1b moves along the axial direction so as to cover the small-diameter cylindrical portion 1a (FIG. 13(b)). At this time, the side surface t1 of the rib 1b1 of the large-diameter cylindrical portion 1b is in contact with the rear edge surface of the flange portion 2b1 of the syringe barrel 2b, so that the syringe barrel 2b also moves in the same direction. Then, the tip of the injection needle 2a penetrates through the needle cap 2d and the seal member 3a to reach the inside of the container 3.

At the same time, the holding member 4 that fixes and supports the syringe barrel 2b is pushed toward the container 3, and as described with reference to FIGS. 11 and 12, the pushing portion 4e of the holding member 4 in a state of sandwiching the flange portion 2d4 of the needle cap 2d is in contact with the edge surface of the container 3, and the tubular portion 2d5 of the front portion 2d3 of the needle cap 2d is pushed against the seal member 3a, and this state is maintained.

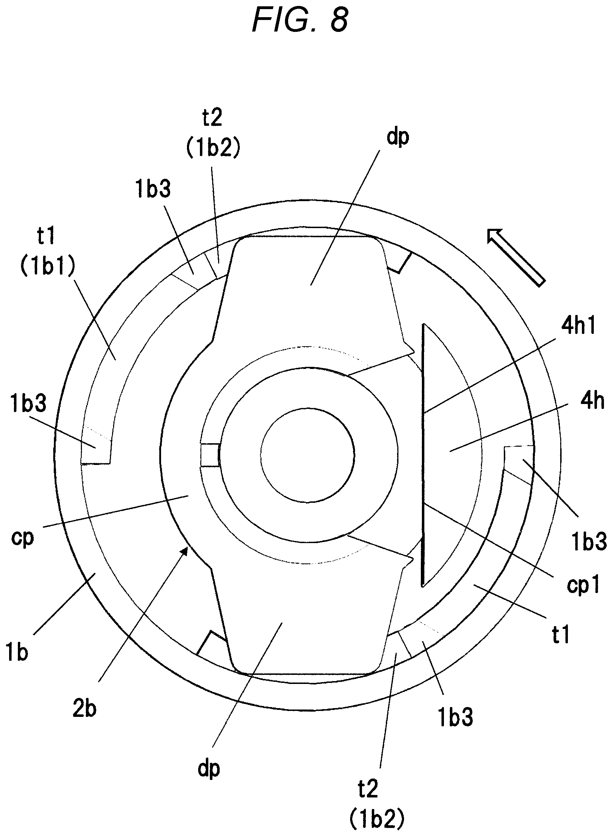

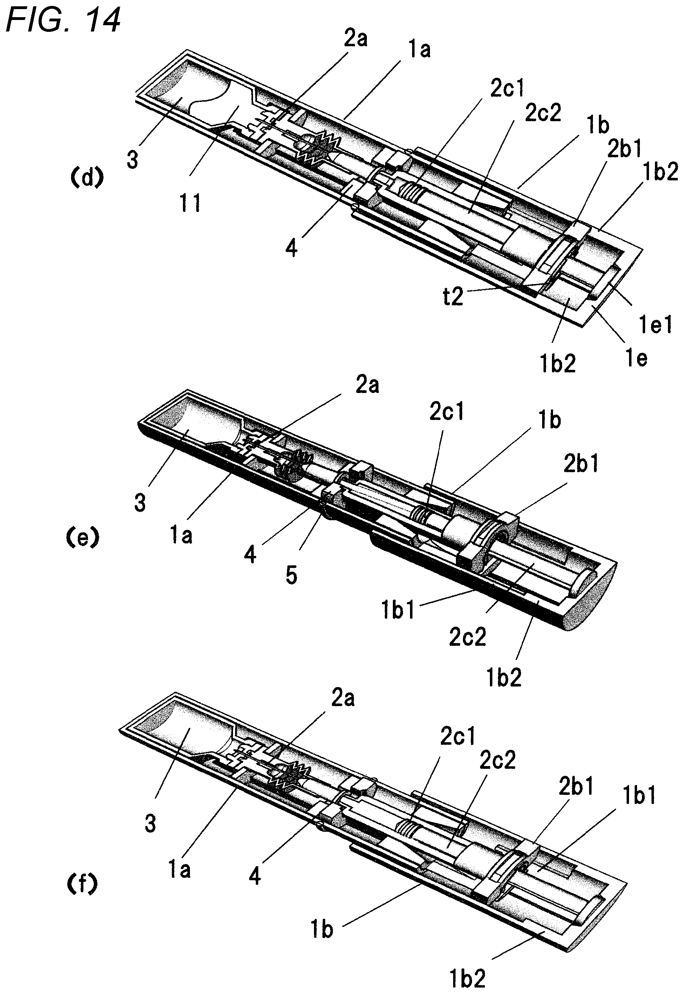

Next, the large-diameter cylindrical portion 1b is slightly pulled back in the axial direction with respect to the small-diameter cylindrical portion 1a, and then the large-diameter cylindrical portion 1b is rotated about 60 degrees to the right direction(in FIG. 8, to left rotation) around the axial center with respect to the syringe barrel 2b as shown in FIG.8, and the two trapezoidal portions dp of the flange portion 2b1 are positioned so as to overlap the second rib 1b2 in the axial direction view (FIG. 13(c)). FIG. 8 is a sectional arrow drawing showing the position of the accommodating case 1 rotated with respect to the syringe barrel 2b. When the large-diameter cylindrical portion 1b is pushed axially into the small-diameter cylindrical portion 1a at the rotational position shown in FIG. 8, the movement is stopped at a position where the side surface t2 of the second rib 1b2 comes into contact with the trapezoidal portion dp of the flange portion 2b1 (FIG. 14(d)). At this time, the driving rod 2c2 that is in contact with the concave portion 1e1 of the wall 1e of the large-diameter cylindrical portion 1b is pushed, and the plunger 2c1 connected to the driving rod 2c2 is pushed into the syringe barrel 2b so that an air in the syringe barrel 2b is injected into the container 3, and the inside of the container 3 becomes in pressurized state.

Subsequently, when the large-diameter cylindrical portion 1b pushing the plunger 2c1 into the syringe barrel 2b is released to free, the pressurized air injected into the container 3 returns to the syringe barrel 2b together with the solution liquid 11 in the container 3, and the plunger 2c1 is pushed back. At the same time, the large-diameter cylindrical portion 1b is also pushed back by the driving rod 2c2 (FIG. 14(e)). Then, the solution liquid 11 that has flowed into the syringe barrel 2b is mixed-type with the powder drug 10 to dissolve it, whereby an injection solution is produced.

Finally, the large-diameter cylindrical portion 1b is slightly pushed in the small-diameter cylindrical portion 1a in the axial direction to move the plunger 2c1, and the bubbles remaining in the syringe barrel 2b are discharged from the tip of the injection needle 2a into the container 3, whereby the preparation of the injection solution is completed (FIG. 14(f)). After that, the large-diameter cylindrical portion 1b is moved to the rear side in the axial direction to be removed from the small-diameter cylindrical portion 1a (Fig. 15(g)), and the syringe 2 is pulled out in the axial direction to be taken out from the holding member 4 (Fig. 15(h)). At this time, since the pushing portion 4e of the holding member 4 pushes the flange portion 2d4 of the needle cap 2d against the edge surface of the container 3 (see FIG. 12), the needle cap 2d fixed-type to the outer circumferential portion of the flange portion 2a1 of the injection needle 2a is peeled off and remains on the holding member 4 side, and the syringe 2 with the injection needle 2a exposed is taken out.

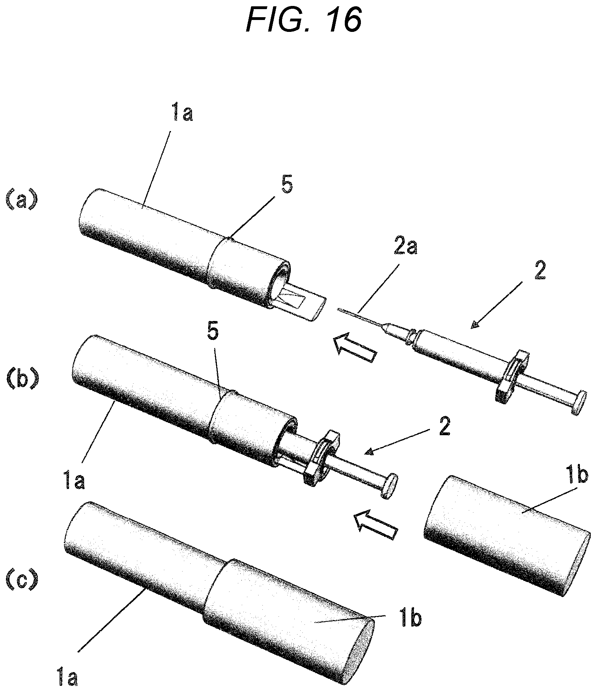

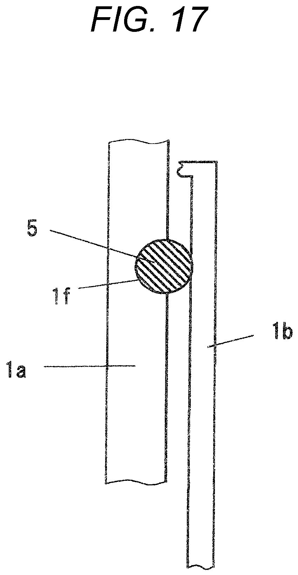

Next, the disposal of the used syringe is described. FIG. 16 is a perspective drawing showing a disposal preparation process of a used syringe, and FIG. 17 is an enlarged schematic sectional drawing of a part of the accommodating case 1 after completion of the disposal preparation process.

First, the used syringe 2 is inserted from the injection needle 2a side into the end opening of the small diameter-cylindrical portion 1a along the axial direction (Fig. 16(a)). Subsequently, the large-diameter cylindrical portion 1b is moved along the axial direction to a position where the large-diameter cylindrical portion 1b completely covers the rear side of the syringe 2 that has popped out from the end opening of the small-diameter cylindrical portion 1a (FIGS. 16(b) and (c)). At this time, as shown in FIG. 17, the large-diameter cylindrical portion 1b is moved to a position where the inner circumferential wall of the large-diameter cylindrical portion 1b is in contact with the O-ring 5 provided on the entire outer circumferential portion of the small-diameter cylindrical portion 1a. As described with reference to FIG. 6, since the protrusion amount W1 of the O-ring 5 from the outer circumferential surface of the small-diameter cylindrical portion 1a is larger than the difference W2 of the positions in the radial direction between the outer circumferential surface of the small-diameter cylindrical portion 1a and the inner circumferential surface of the large-diameter cylindrical portion 1b, the O-ring 5 is crushed evenly over the entire circumference, and the inside of the accommodating case 1 is sealed from the outside by the O-ring 5. As a result, the injection solution remaining inside the syringe 2 does not leak outside.

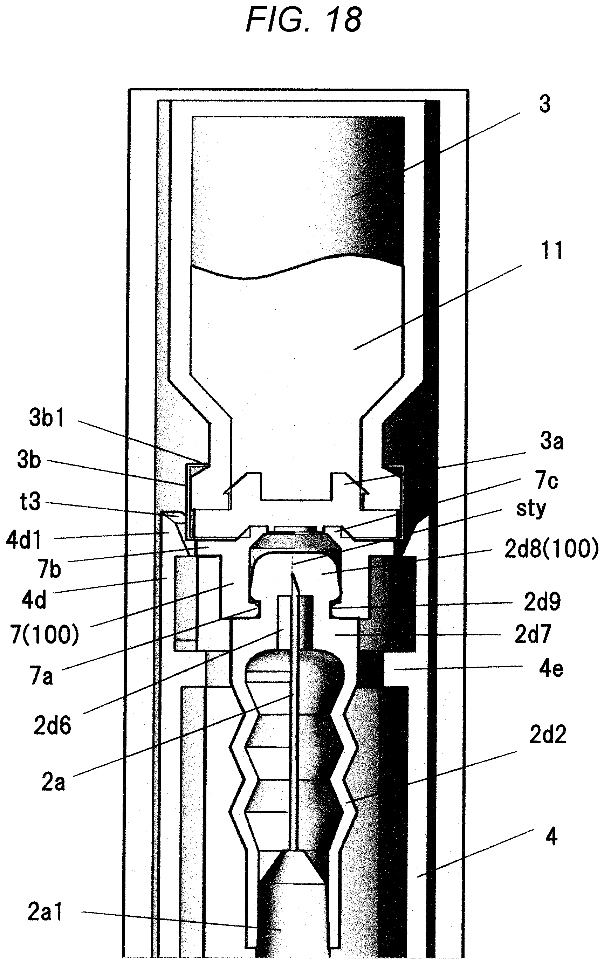

Next, another embodiment of the two-component-mixing prefilled syringe kit according to the present invention is described. FIG. 18 is a partially enlarged cross-sectional drawing of the prefilled syringe kit according to the first another embodiment of the present invention.

In this another embodiment, a cylindrical rubber adapter 7 is attached to the surface of the seal member 3a, and an insertion portion 2d8 that can be fitted into the inner surface portion of the adapter 7 to be coupled to it is provided at the front portion 2d7 of the needle cap 2d and the difference is that the adapter 7 and the insertion portion 2d8 form a space sealed from the outside on the surface side of the seal member 3a. That is, the space-forming body 100 is configured by the adapter 7 and the insertion portion 2d8 of the needle cap 2d.

The adapter 7 has a toroidal protrusion 7a protruding inward at the end on the needle cap 2d side, and has a disk-shaped flange portion 7b protruding outward and a cylindrical leg portion 7c inclining inward toward the surface of the seal member 3a at the end on the container 3 side. The insertion portion 2d8 of the needle cap 2d is formed in a tapered columnar shape whose outer diameter decreases toward the tip, and has a groove portion 2d9 having a diameter smaller than that of the protrusion 7a on the base side. As a result, the insertion portion 2d8 is pushed into close contact with the inner surface of the adapter 7, and the protrusion 7a of the adapter 7 is fitted into the groove portion 2d9 of the insertion portion 2d8 so as to prevent the insertion portion 2d8 from coming off the adapter 7.

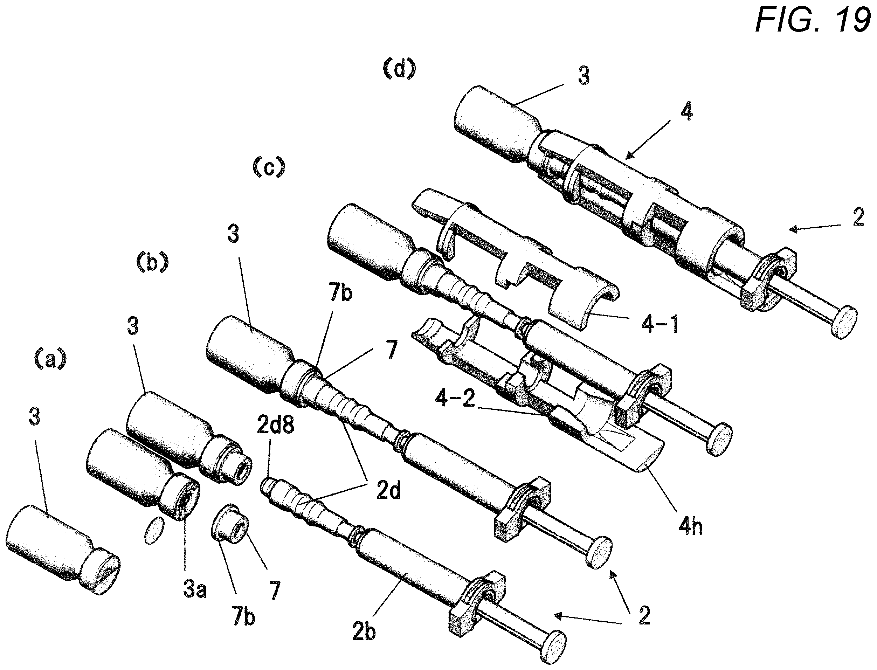

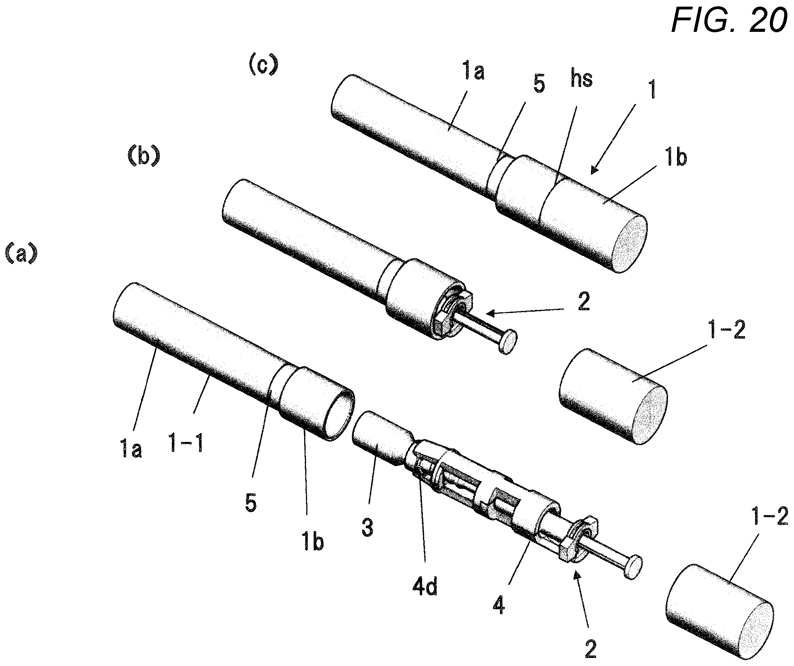

Next, the assembly of the two-component-mixing prefilled syringe kit of the first another embodiment is described. FIG.19 is a perspective drawing showing an assembling process of the syringe to the holding member, and FIG. 20 is a perspective drawing showing an assembling process of the prefilled syringe kit shown in FIG. 18.

In assembling the syringe 2 to the holding member 4, first, the protective film of the seal member 3a of the container 3 is removed, and the adapter 7 is temporarily fixed-type to the surface of the seal member 3a with an adhesive (FIG. 19(a)). At this time, the outer side surface of the leg portion 7c of the adapter 7 closely faces the surface of the seal member 3a of the container 3. Next, the insertion portion 2d8 at the tip of the needle cap 2d of the syringe 2 in which the powder drug 10 is filled in the syringe barrel 2b in advance is inserted into the adapter 7 to integrally connect the container 3 and the syringe 2 (FIG. 19(b)). ). Next, the syringe 2 to which the container 3 is coupled is set from above on the semi-cylindrical member 4-2 with the inside facing upward. At this time, the position is set so that the straight outer circumferential portion cp1 of the flange portion 2b1 comes into contact with the rotation stop portion 4h of the holding member 4 (see FIG. 7). Also, the semi-cylindrical member 4-1 with the inside facing downward is covered over the syringe 2 to which the container 3 is coupled, and is integrally coupled with the semi-cylindrical member 4-2 (FIGS. 9(c) and (d)). As a result, the holding member 4 is formed by the semi-cylindrical members 4-1 and 4-2 that are integrally connected, and the syringe 2 connected to the container 3 is assembled to the holding member 4.

In assembling the prefilled syringe kit, the syringe 2 assembled to the holding member 4 is inserted into the end opening of the case component 1-1 on the large-diameter cylindrical portion 1b side with the container 3 side front (FIG. 20(a)). Next, the case component 1-2 is covered so as to cover the rear side of the syringe 2 protruding from the opening of the case component 1-1, and the opening ends of both the case components 1-1 and 1-2 are joined by that both step portions 1b4 and 1b5 are fitted to each other (FIGS. 20(b) and (c)). Finally, the step portions 1b4 and 1b5 of the two case components 1-1 and 1-2 joined are joined by heat to form a joint hs, and the inside of the case 1 is sealed.

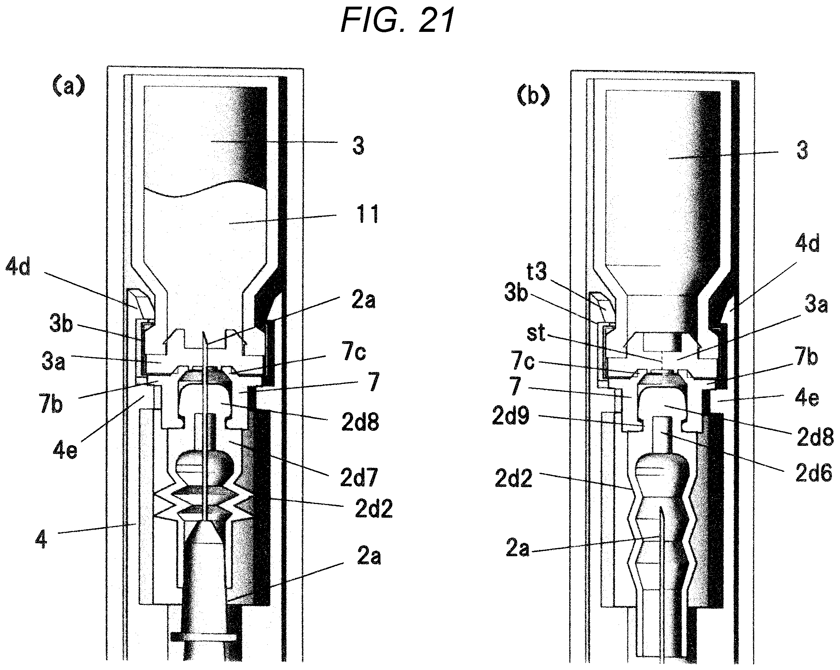

Next, the injection solution preparation operation by the two-component-mixing prefilled syringe kit of the first another embodiment is described. FIG. 21 is a sectional drawing for explaining an injection solution preparing operation by the prefilled syringe kit of FIG.18

When the syringe barrel 2b is pushed into the container 3 side, the bellows portion 2d2 of the needle cap 2d contracts, and the injection needle 2a pierced up to the midpoint of the insertion portion 2d8 of the needle cap 2d penetrates through the remaining portion of the insertion portion 2d8 and the seal member 3a and projects into the inside of the container 3 (FIGS. 18 and FOG. 21(a)). At this time, the holding member 4 holding the syringe barrel 2b is also pushed toward the container 3 side, and the hook portion 4d1 of the movable piece 4d that is in contact with the circumferential edge corner of the stopper 3b of the container 3 at the inclined surface t3 slides outward, as it moves, the support portion 4d2 bends outward, and the hook portion 4d1 that has moved outward passes by sliding on the side surface of the stopper 3b. At the same time, the pushing portion 4e of the holding member 4 pushes the flange portion 7b of the adapter 7 against the front surface of the stopper 3b of the container 3. Then, the hook portion 4d1 pushed out to the outside returns to the inside and is hanged by the step portion 3b1 on the base side of the stopper 3b, and the holding member 4 is mechanically coupled to the container 3 at this position. As a result, the state in which the injection needle 2a projects into the container 3 and the state in which the flange portion 7b of the adapter 7 is pushed against the front surface of the stopper 3b of the container 3 are maintained.

Next, the plunger 2c1 is pushed with a finger to send the air in the syringe barrel 2b into the container 3 to increase the pressure in the container 3, and then the finger is released from the plunger 2c1 to be free, the liquid 11 in the container 3 is suctioned to the inside of the syringe barrel 2b through the injection needle 2a by internal pressure, mixed-type with the powdered drug 10 inside the syringe barrel 2b to produce an injection solution, and the plunger 2c1 is returned to its original position. Finally, the syringe barrel 2b is grasped with fingers, the injection needle 2a is pulled out from the container 3, and is pulled back to the inside of the needle cap 2d (FIG. 21(b)). At this time, a part of the liquid 11 remaining in the container 3 may overflow onto the surface of the seal member 3a through the needle piercing point st of the seal member 3a (see FIG. 12), but, since the space surrounded by the seal member 3a and the adapter 7 and the insertion portion 2d8 of the cap 2d is sealed from the outside, the liquid 11a overflowing from the container 3 does not leak to the outside.

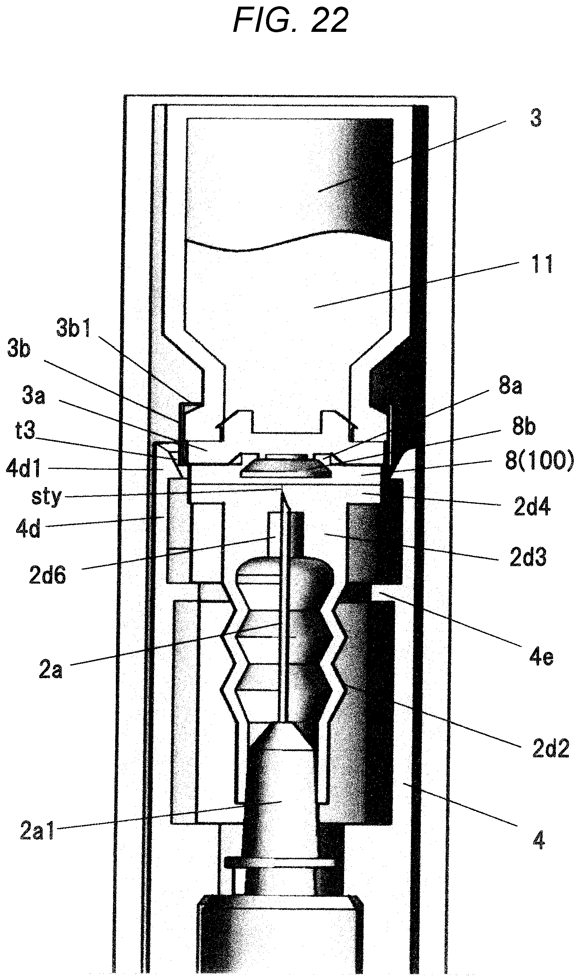

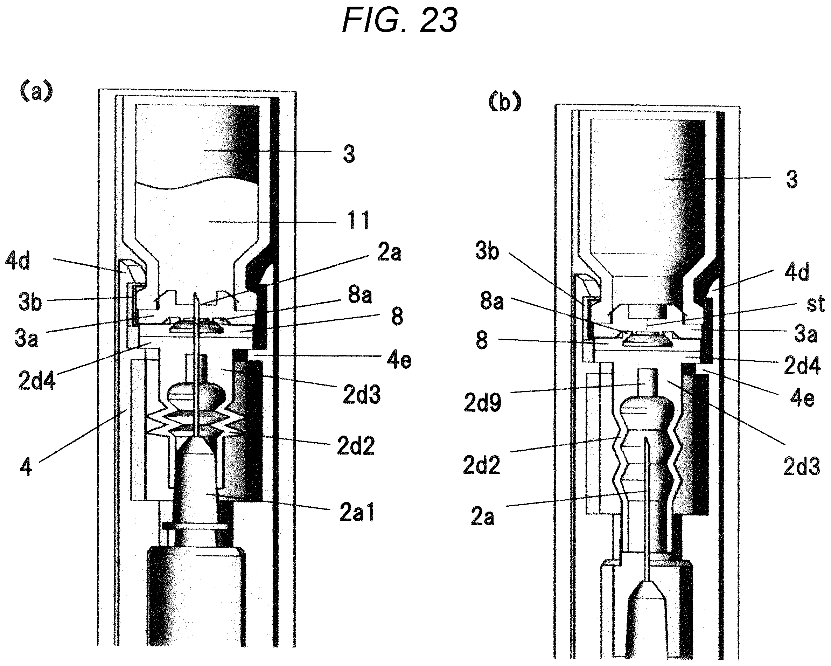

Next, a second another embodiment of the two-component-mixing prefilled syringe kit of the present invention is described. FIG. 22 is a partially enlarged cross-sectional drawing of the prefilled syringe kit according to the second another embodiment of the present invention, and FIG. 23 is a cross-sectional drawing for explaining an injection solution preparing operation by the prefilled syringe kit of FIG.22.

In the second another embodiment, the difference is that a disk-shaped rubber adapter 8 attached to the surface of the seal member 3a has a cavity 8b opened only on the seal member 3a side, and the adapter 8 is attached to the surface of the seal member 3a, whereby a space sealed from the outside is formed on the surface side of the seal member 3a. That is, the space-forming body 100 is configured by the adapter 8.

The adapter 8 is formed flat plate-like at the edge portion on the needle cap 2d side, and has a cylindrical leg portion 8a inclined inward toward the surface of the seal member 3a at the edge portion on the seal member 3a side. The needle cap 2d has a front portion 2d3 whose side facing the adapter 8 is formed a flat surface, and the front portion 2d3 is provided with an annular plate-shaped flange portion 2d4.

Next, the injection solution preparation operation by the two-component-mixing prefilled syringe kit of another embodiment is described. FIG. 23 is a cross-sectional drawing for explaining an injection solution preparing operation by the prefilled syringe kit of FIG.22.

When the syringe barrel 2b is pushed into the container 3 side, the bellows portion 2d2 of the needle cap 2d contracts, and the injection needle 2a pierced up to the middle of the front portion 2d3 of the needle cap 2d penetrates the remaining part of the front portion 2d3, the adapter 8 and the seal member 3a to project into the container 3 (FIGS. 22 and FIG. 23(a)). At this time, the holding member 4 holding the syringe barrel 2b is also pushed toward the container 3 side, and the hook portion 4d1 of the movable piece 4d that is in contact with the circumferential edge corner of the stopper 3b of the container 3 on the inclined surface t3 slides outward, as it moves, the support portion 4d2 bends outward, and the hook portion 4d1 that has moved outward passes by sliding on the side surface of the stopper 3b. At this time, the pushing portion 4e of the holding member 4 overlaps the flange portion 2d4 of the needle cap 2d and the circumferential edge portion of the disk-shaped adapter 8 and pushes them against the front surface of the stopper 3b of the container 3. Then, the hook portion 4d1 pushed out to the outside returns to the inside and is hanged by the step portion 3b1 on the base side of the stopper 3b, and the holding member 4 is mechanically coupled to the container 3 at this position. As a result, the state in which the injection needle 2a is projected into the container 3 and the state in which the pushing portion 4e of the holding member 4 overlaps the flange portion 2d4 of the needle cap 2d and the circumferential edge portion of the disk-shaped adapter 8 to push them against the front surface of the stopper 3b of the container 3 are maintained.

Next, the plunger 2c1 is pushed with a finger to send the air in the syringe barrel 2b into the container 3 to increase the pressure in the container 3, and then release the finger from the plunger 2c1 to be free, the liquid 11 in the container 3 is suctioned to the inside of the syringe barrel 2b through the injection needle 2a by internal pressure, and is mixed-type with the powder drug 10 inside the syringe barrel 2b to produce an injection solution, and the plunger 2c1 is returned to its original position. Finally, the syringe barrel 2b is grasped with fingers so that the injection needle 2a is pulled out from the container 3, and is pulled back to the inside of the needle cap 2d (FIG. 23(b)). At this time, a part of the liquid 11a of the liquid 11 remaining in the container 3 may overflow to the surface of the seal member 3a through the needle piercing portion st of the seal member 3a (see FIG. 12). But, since the cavity 8b of the adapter 8 communicating with the surface of the seal member 3a is sealed from the outside, the liquid 11a overflowing from the container 3 does not leak to the outside.

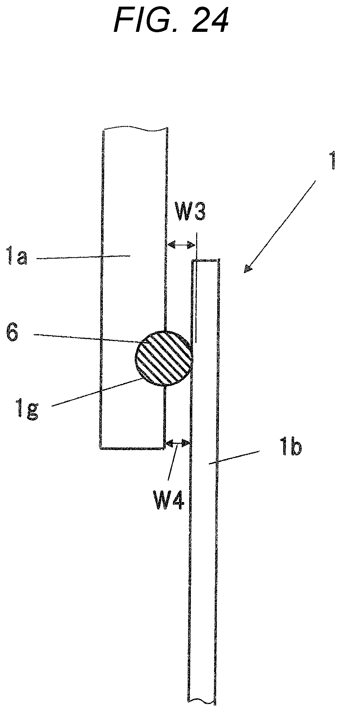

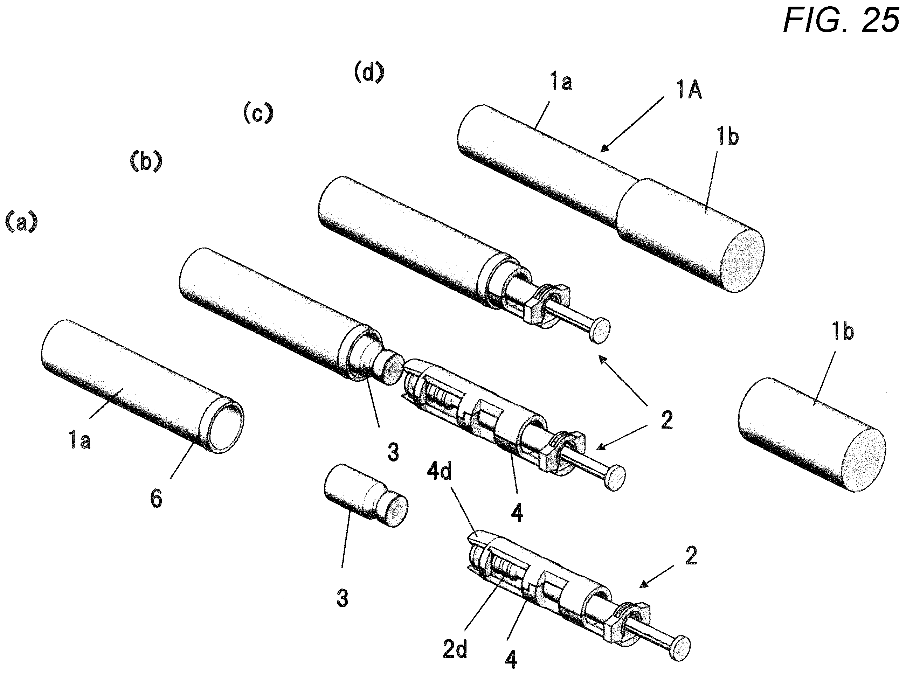

Next, a third another embodiment of the two-component-mixing prefilled syringe kit according to the present invention is described. FIG. 24 is a partially enlarged cross-sectional drawing showing the structure of a prefilled syringe kit according to a third another embodiment of the present invention, and FIG. 25 is a perspective drawing showing an assembly process of the prefilled syringe kit shown in FIG.24.

In the third another embodiment, the structure of the accommodating case 1A is different from that of the above-described prefilled syringe kit, and the other structures are the same.

The accommodating case 1A does not have a structure in which the small-diameter cylindrical portion 1a and the large-diameter cylindrical portion 1b are connected by the thin plate portion 1c as in the above-described embodiment, but has a structure in which in a state where the small-diameter cylindrical portion 1a and the large-diameter cylindrical portion 1b are mutually axially aligned, the O-ring 6 is arranged between the outer circumferential portion of one end of the small-diameter cylindrical portion 1a and the inner circumferential portion of one end of the large-diameter cylindrical portion 1b.

A concave groove 1g having a semicircular cross section is formed over the entire circumference at the outer circumference of the small-diameter cylindrical portion 1a, and an O-ring 6 is fitted in the concave groove 1g. The protrusion amount W3 of the O-ring 6 from the outer circumferential surface of the small-diameter cylindrical portion 1a is set to a value larger than half of the difference W4 between the inner diameter of the large-diameter cylindrical portion 1b and the outer diameter of the small-diameter cylindrical portion 1a (W3 >W4). Therefore, the O-ring 6 is crushed evenly over the entire circumference, and the inside of the accommodating case 1A is sealed from the outside. Also, the concave groove 1g is provided closer to one end of the small-diameter cylindrical portion 1a than the concave groove 1f of the above embodiment.

In the process of assembling the prefilled syringe of the third another embodiment, first, the container 3 is inserted into the end opening of the small-diameter cylindrical portion 1a from the bottom side (FIG. 25(a)). Subsequently, the holding member 4 set with the syringe 2 is inserted into the small-diameter cylindrical portion 1a with the movable piece 4d facing forward (FIG. 25(b)). Next, the large-diameter cylindrical portion 1b is covered so as to cover the rear side of the syringe 2 that has protruded from the end opening of the small-diameter cylindrical portion 1a (Fig. 18(c)), and the large-diameter cylindrical portion 1b is moved in the axial direction to a position where the inner circumferential surface of the large-diameter cylindrical portion 1b is in contact with the O-ringed 6, so that to form a sealed accommodating case 1A (FIG. 18(d)).

In the use of the prefilled syringe of the third another embodiment, since the small-diameter cylindrical portion 1a and the large-diameter cylindrical portion 1b are not directly connected, except the step of first breaking and separating the small-diameter cylindrical portion 1a and the large-diameter cylindrical portion 1b is unnecessary, it is the same as the above-described embodiment except that

The disposal of the used syringe by the prefilled syringe of the third another embodiment is the same as in the above embodiment (see FIG. 14). When the used syringe 2 is stored in the accommodating case 1A in another embodiment, the inner circumferential wall of the large-diameter cylindrical portion 1b is in contact with the O-ringed 6 provided on the entire outer circumference of the small-diameter cylindrical portion 1a, whereby the inside of the accommodating case 1A is sealed from the outside.

Next, another embodiment according to the present invention is described.

First, in the above-described embodiment, the powder drug 10 is filled in the inside of the syringe barrel 2b as one component constituting the injection solution, and the liquid 11 (dissolved solution or dispersion) is filled in the inside of the container 3 as the other component constituting the injection solution. However, the syringe barrel 2b may be filled with a solid drug such as granules instead of the powder drug.

Also, both the syringe barrel 2b and the container 3 may be filled with the liquid component. Specifically, the container 3 is filled with a liquid drug such as a vaccine, which has poor stability when premixed-type, and the syringe barrel 2b is filled with the solution. Alternatively, the syringe barrel 2b is filled with the liquid drug and the container 3 is filled with the solution. As a result, it is effective when it is necessary to separately fill a liquid drug having poor stability with a solution and to mix them when preparing an injection solution.

In the above-described embodiment, the tip of the injection needle 2a is held in a state of being pierced by the needle cap 2d (however, in a non-penetrating state), but it is also good that the tip of the injection needle 2a is held in a state of being separated from the needle cap 2d.

In the above-described embodiment, the holding member 4 holds the syringe barrel 2b so as to be movable along the barrel axis direction of the syringe barrel 2b, but it may be configured that the holding member holds the container 3 so as to be movable along the barrel axis direction of the syringe barrel 2b.

In the above-described embodiment, the container 3 is configured by a vial, but may be configured by a container other than.

The embodiments disclosed this time are to be considered as illustrative in all points and not restrictive. The scope of the present invention is shown not by the above description but by the scope of the claims.

### Explanation of symbols

1 accommodating case, 1A accommodating case, 1a small-diameter cylindrical portion, 1b large-diameter cylindrical portion, 1b1 rib, 1b2 second rib, 1b3 third rib, 1b4 step portion, 1b5 step portion, 1c thin plate portion, 1d wall, 1e wall, 1e1 concave portion,
1f concave groove, 1g concave groove, 1-1 case component, 1-2 case component, 2 syringe, 2a injection needle, 2a1 flange portion, 2b syringe barrel, 2b1 flange portion, 2c1 plunger, 2c2 driving rod, 2d needle cap, 2d1 cylindrical portion, 2d2 bellows portion, 2d3 front portion, 2d4 flange portion, 2d5 tubular portion, 2d6 cavity, 2d7 front portion, 2d8 insertion portion, 2d9 groove portion, D height, H inner diameter, V space (closed space), 3 container,
3a seal member, 3b stopper, 3b1 step portion, 3c bottom portion, 3d flange portion, 4 holding member, 4a toroidal rib,
4b toroidal step portion, 4c cylindrical guide, 4d movable piece, 4d1 hook portion (lock part, claw body),
4d2 support portion, 4e pushing portion, 4a1 step portion, 4a2 step portion, 4e1 convex portion, 4e2 step portion, 4f cylindrical guide, 4-1 Semi-cylindrical member, 4-2 Semi-cylindrical member, 5 O-ring, 6 O-ring, 7 adapter, 7a protrusion portion, 7b flange portion, 7c leg portion, 8 adapter, 8a leg portion, 8b cavity, 10 powder drug, 11 liquid (dissolution, dispersion), 50 mount portion, 100 space-forming body, Hs joint, St needle piercing point, Sty expected piercing point, t1 side surface, t2 side surface, t3 inclined surface

## Claims

1. A two-component-mixing prefilled syringe kit, comprising;
A syringe barrel (2b) in which an injection needle (2a) is attached to one end, a plunger is slidably inserted from an opening at the other end, and a solid component or a liquid component constituting an injection solution is filled inside;
A container (3) in which an outlet is sealed with a seal member (3a) that can be pierced by the injection needle (2a), and a liquid component constituting the injection solution is filled inside;
A holding member (4) for holding at least one of the syringe barrel (2b) or the container (3) so as to be movable along the barrel axis direction of the syringe barrel (2b) with the tip of the injection needle (2a) facing the outlet of the container (3);
A needle cap (2d) which is formed of a material that can be pierced by the injection needle (2a) to cover the injection needle (2a) from the tip side, and fixed at base end side to a base portion of the injection needle (2a) or an outer surface of the syringe barrel (2b);Wherein, a space-forming body (100) which forms a space facing the surface of the seal member (3a) with a space sealed from the outside is arranged so as to be in close contact with the surface of the seal member (3a), and the space-forming body (100) is formed of a material which can be pierced by the injection needle (2a) at a portion facing the tip of the injection needle (2a) along the barrel axis direction of the syringe barrel (2b); and
wherein;
The needle cap (2d) has a tubular portion (2d5) in which a tubular axis direction is along to the direction toward the seal member (3a) and an expected piercing point by the injection needle (2a) is located at the inner side, on a front portion facing the seal member (3a),
The space-forming body (100) is constituted by the front portion of the needle cap (2d) and the tubular portion (2d5) in close contact with the surface of the seal member (3a);
**characterized in that**
The needle cap (2d) has a flange portion(2d4) being in a direction intersecting with the barrel axis direction of the syringe barrel (2b) on the outer side surface of the front portion; and
The holding member (4) comprises a mount portion (50) for mounting the syringe barrel (2b), a pushing portion (4e) for pushing the flange portion (2d4) against the container (3) when moved to the container side along the barrel axis direction of the syringe barrel (2b), and a lock part for holding a state where the pushing portion (4e) pushes the flange portion (2d4) against the container (3).

2. The two-component-mixing prefilled syringe kit as defined in claim 1, wherein;
The length of the tubular portion (2d5) along the tubular axis direction is less than 4 mm.

3. A two-component-mixing prefilled syringe kit, comprising;
A syringe barrel (2b) in which an injection needle (2a) is attached to one end, a plunger is slidably inserted from an opening at the other end, and a solid component or a liquid component constituting an injection solution is filled inside;
A container (3) in which an outlet is sealed with a seal member (3a) that can be pierced by the injection needle (2a), and a liquid component constituting the injection solution is filled inside;
A holding member (4) for holding at least one of the syringe barrel (2b) or the container (3) so as to be movable along the barrel axis direction of the syringe barrel (2b) with the tip of the injection needle (2a) facing the outlet of the container (3);
A needle cap (2d) which is formed of a material that can be pierced by the injection needle (2a) to cover the injection needle (2a) from the tip side, and fixed at the base end side to a base portion of the injection needle (2d) or the outer surface of the syringe barrel (2b);
A tubular container adapter (7) which has a tube axis coinciding with the barrel axis direction of the syringe barrel (2b), and fixed in close contact with the surface of the seal member (3a) at an one end in the tube axis direction;
The needle cap (2d) has an insertion portion (2d8) inserted along the tube axis direction of the container adapter from the other end side in the tube axis direction so as to be in close contact with the inner surface of the container adapter, at a front portion facing the seal member (3a); **characterized in that**
a space-forming body (100) which forms a space facing the surface of the seal member with a space sealed from the outside is constituted by the container adapter (7) and the insertion portion (2d8) of the needle cap (2d), and the space forming body (100) is formed of a material which can be pierced by the injection needle (2a) at a portion facing the tip of the injection needle (2a) along the barrel axis direction of the syringe barrel (2b).

4. The two-component-mixing prefilled syringe kit as defined in claim 3, wherein;
The container adapter (7) has a flange portion (7b) being in a direction intersecting with the barrel axis direction of the syringe barrel (2b), on the outer side surface of the seal member side,
The holding member (4) has a mount portion (50) for mounting the syringe barrel (2b), and a pushing portion (4e) for pushing the flange portion (7b) against the container (3) when the syringe barrel (2b) mounted on the mount portion (50) is moved along a barrel axis direction to the container side, and a lock part for holding the state where the pushing portion (4e) pushes the flange portion (7b) against the container (3).

5. A two-component-mixing prefilled syringe kit, comprising;
A syringe barrel (2b) in which an injection needle (2a) is attached to one end, a plunger is slidably inserted from an opening at the other end, and a solid component or a liquid component constituting an injection solution is filled inside;
A container (3) in which an outlet is sealed with a seal member (3a) that can be pierced by the injection needle (2a), and a liquid component constituting the injection solution is filled inside;
A holding member (4) for holding at least one of the syringe barrel (2b) or the container (3) so as to be movable along the barrel axis direction of the syringe barrel (2b) with the tip of the injection needle (2a) facing the outlet of the container (3);
A needle cap (2d) which is formed of a material that can be pierced by the injection needle (2a) to cover the injection needle (2a) from the tip side, and fixed at a base end side to a base portion of the injection needle (2a) or the outer surface of the syringe barrel (2b);
A container adapter (8) having a tubular portion (8a) which is fixed in close contact with the surface of the seal member (3a) at one end in the tube axial direction, and a flat plate-shaped main body portion which covers the opening on the other end side of the tubular portion (8a);
The main body portion of the container adapter is formed of a material pierceable by the injection needle (2a) at a portion facing the seal member (3a), **characterized in that**
a space-forming body (100) which forms a space facing the surface of the seal member with a space sealed from the outside is constituted by the container adapter (8), and the space forming body (100) is formed of a material which can be pierced by the injection needle (2a) at a portion facing the tip of the injection needle (2a) along the barrel axis direction of the syringe barrel (2b).

6. The two-component-mixing prefilled syringe kit as defined in claim 5, wherein;
The container adapter (8) has a flange portion being in a direction intersecting with the barrel axis direction of the syringe barrel (2b) on the outer surface of the seal member side,
The needle cap (2d) has a flange portion (2d4) being in a direction intersecting with the barrel axis direction of the syringe barrel (2b) on the outer side surface of the front side,
The holding member (4) has a mount portion (50) for mounting the syringe barrel (2b), and a pushing portion (4e) that pushes the two flange portions (2d4) of the needle cap (2d) and the container adapter (8) in overlapped state against the container (3) when moving to the container side along the barrel axis direction of the syringe barrel (2b) and the lock part that holds the state where the pushing portion (4e) pushes the two flange portions (2d4) against the container (3).

7. The two-component-mixing prefilled syringe kit as defined in claim 5 or 6, wherein;
The length of the tubular portion (8a) along the tube axis direction is less than 4 mm.

8. The two-component mixing prefilled syringe kit as defined in any of claim 1 to 7, wherein;
A volume of space surrounded by the surface of the seal member (3a) and the space-forming body (100) is in the range of 0.01 ml to 0.03 ml.

9. The two-component-mixing prefilled syringe kit as defined in any of claim 1, 4 or 6, wherein;
It is characterized that the lock part is a claw body which can be engaged with a concave portion or a step portion (3b1) on the outer surface of the container (3).

10. The two-component-mixing prefilled syringe kit as defined in claim 1, wherein;
comprises an accommodating case (1) which accommodates the whole of the syringe barrel (2b), the needle cap (2d), the container (3), and the holding member (4) in a sealed state to the outside.

11. The two-component-mixing prefilled syringe kit as defined in claim any of 3 to 7, wherein;
comprises an accommodating case (1) which accommodates the whole of the syringe barrel (2b), the needle cap (2d), the container adapter (7, 8), the container (3) and the holding member (4) in a sealed state to the outside.

12. The two-component-mixing prefilled syringe kit as defined in claim any of 1 to 11, wherein;
The tip of the injection needle (2a) is held by the needle cap (2d) in a non-penetrated piecing state.

## Patentansprüche

1. Mit einer Zweikomponentenmischung vorgefülltes Spritzenset, umfassend:
Einen Spritzenzylinder (2b), in dem eine Injektionsnadel (2a) an einem Ende angebracht ist, ein Kolben schiebbar von einer Öffnung an dem anderen Ende eingeführt ist und eine feste Komponente oder eine flüssige Komponente, die eine Injektionslösung bildet, ins Innere gefüllt ist;
Einen Behälter (3), in dem ein Auslass mit einem Dichtelement (3a) abgedichtet ist, das von der Injektionsnadel (2a) durchstochen werden kann, und eine flüssige Komponente, welche die Injektionslösung bildet, ins Innere gefüllt ist;
Ein Halteelement (4) zum Halten von mindestens einem aus dem Spritzenzylinder (2b) oder dem Behälter (3) derart, dass er entlang der Zylinderachsenrichtung des Spritzenzylinders (2b) beweglich ist, wobei die Spitze der Injektionsnadel (2a) dem Auslass des Behälters (3) zugewandt ist;
Eine Nadelkappe (2d), die aus einem Material, das von der Injektionsnadel (2a) durchstochen werden kann, ausgebildet ist, um die Injektionsnadel (2a) von der Spitzenseite zu bedecken, und die an einer Basisendseite an einem Basisabschnitt der Injektionsnadel (2a) oder an einer Außenfläche des Spritzenzylinders (2b) befestigt ist; wobei ein raumausbildender Körper (100), der einen der Fläche des Dichtelements (3a) zugewandten Raum mit einem zur Außenseite abgedichteten Raum ausbildet, derart angeordnet ist, dass er in engem Kontakt mit der Fläche des Dichtelements (3a) steht, und der raumausbildende Körper (100) aus einem Material ausgebildet ist, das von der Injektionsnadel (2a) an einem Abschnitt durchstochen werden kann, welcher der Spitze der Injektionsnadel (2a) entlang der Zylinderachsenrichtung des Spritzenzylinders (2b) zugewandt ist; und
wobei;
Die Nadelkappe (2d) auf einem dem Dichtelement (3a) zugewandten Abschnitt einen rohrförmigen Abschnitt (2d5) aufweist, in dem eine rohrförmige Achsenrichtung längs zu der Richtung zum Dichtelement (3a) hin verläuft und sich auf der Innenseite ein von der Injektionsnadel (2a) vorgesehener Durchstechpunkt befindet,
Der raumausbildende Körper (100) durch den vorderen Abschnitt der Nadelkappe (2d) gebildet ist und der rohrförmige Abschnitt (2d5) in engem Kontakt mit der Oberfläche des Dichtelements (3a) ist;
**gekennzeichnet dadurch, dass**
Die Nadelkappe (2d) auf der Außenseitenfläche des vorderen Abschnitts einen Flanschabschnitt (2d4) aufweist, der in eine Richtung verläuft, welche die Zylinderachsenrichtung des Spritzenzylinders (2b) schneidet; und
Das Halteelement (4) Folgendes umfasst: einen Montageabschnitt (50) zum Montieren des Spritzenzylinders (2b), einen Schiebeabschnitt (4e) zum Schieben des Flanschabschnitts (2d4) gegen den Behälter (3), wenn er entlang der Zylinderachsenrichtung des Spritzenzylinders (2b) zu der Behälterseite bewegt wird, und einen Verriegelungsteil zum Halten eines Zustands, in dem der Schiebeabschnitt (4e) den Flanschabschnitt (2d4) gegen den Behälter (3) schiebt.

2. Mit einer Zweikomponentenmischung vorgefülltes Spritzenset wie in Anspruch 1 definiert, wobei:
Die Länge des rohrförmigen Abschnitts (2d5) entlang der rohrförmigen Achsenrichtung geringer als 4 mm ist.

3. Mit einer Zweikomponentenmischung vorgefülltes Spritzenset, umfassend:
Einen Spritzenzylinder (2b), in dem eine Injektionsnadel (2a) an einem Ende angebracht ist, ein Kolben schiebbar von einer Öffnung an dem anderen Ende eingeführt ist und eine feste Komponente oder eine flüssige Komponente, die eine Injektionslösung bildet, ins Innere gefüllt ist;
Einen Behälter (3), in dem ein Auslass mit einem Dichtelement (3a) abgedichtet ist, das von der Injektionsnadel (2a) durchstochen werden kann, und eine flüssige Komponente, welche die Injektionslösung bildet, ins Innere gefüllt ist;
Ein Halteelement (4) zum Halten von mindestens einem aus dem Spritzenzylinder (2b) oder dem Behälter (3) derart, dass er entlang der Zylinderachsenrichtung des Spritzenzylinders (2b) beweglich ist, wobei die Spitze der Injektionsnadel (2a) dem Auslass des Behälters (3) zugewandt ist;
Eine Nadelkappe (2d), die aus einem Material, das von der Injektionsnadel (2a) durchstochen werden kann, ausgebildet ist, um die Injektionsnadel (2a) von der Spitzenseite zu bedecken, und die an der Basisendseite an einem Basisabschnitt der Injektionsnadel (2a) oder an einer Außenfläche des Spritzenzylinders (2b) befestigt ist;
Einen rohrförmigen Behälteradapter (7), der eine Rohrachse aufweist, die mit der Zylinderachsenrichtung des Spritzenzylinders (2b) zusammenfällt, und in engem Kontakt mit der Fläche des Dichtelements (3a) an einen Ende in der Rohrachsenrichtung befestigt ist;
Die Nadelkappe (2d) weist an einem dem Dichtelement zugewandten vorderen Abschnitt einen Einführungsabschnitt (2d8) auf, der von der anderen Endseite in der Rohrachsenrichtung entlang der Rohrachsenrichtung des Behälteradapters derart eingeführt ist, dass er in engem Kontakt mit der Innenfläche des Behälteradapters ist;
**gekennzeichnet dadurch, dass**
ein raumausbildender Körper (100), der einen der Fläche des Dichtelements zugewandten Raum mit einem zur Außenseite abgedichteten Raum ausbildet, von dem Behälteradapter (7) und dem Einführungsabschnitt (2d8) der Nadelkappe (2d) gebildet ist, und der raumausbildende Körper (100) an einem der Spitze der Injektionsnadel (2a) entlang der Zylinderachsenrichtung des Spritzenzylinders (2b) zugewandten Abschnitt aus einem Material ausgebildet ist, das von der Injektionsnadel (2a) durchstochen werden kann.

4. Mit einer Zweikomponentenmischung vorgefülltes Spritzenset wie in Anspruch 3 definiert, wobei:
Der Behälteradapter (7) auf der Außenseitenfläche der Dichtelementseite einen Flanschabschnitt (7b) aufweist, der in eine Richtung verläuft, welche die Zylinderachsenrichtung des Spritzenzylinders (2b) schneidet,
Das Halteelement (4) Folgendes aufweist: einen Montageabschnitt (50) zum Montieren des Spritzenzylinders (2b) und einen Schiebeabschnitt (4e) zum Schieben des Flanschabschnitts (7b) gegen den Behälter (3), wenn der auf den Montageabschnitt (50) montierte Spritzenzylinder (2b) entlang einer Zylinderachsenrichtung zu der Behälterseite bewegt wird, und einen Verriegelungsteil zum Halten eines Zustands, in dem der Schiebeabschnitt (4e) den Flanschabschnitt (7b) gegen den Behälter (3) schiebt.

5. Mit einer Zweikomponentenmischung vorgefülltes Spritzenset, umfassend:
Einen Spritzenzylinder (2b), in dem eine Injektionsnadel (2a) an einem Ende angebracht ist, ein Kolben schiebbar von einer Öffnung an dem anderen Ende eingeführt ist und eine feste Komponente oder eine flüssige Komponente, die eine Injektionslösung bildet, ins Innere gefüllt ist;
Einen Behälter (3), in dem ein Auslass mit einem Dichtelement (3a) abgedichtet ist, das von der Injektionsnadel (2a) durchstochen werden kann, und eine flüssige Komponente, welche die Injektionslösung bildet, ins Innere gefüllt ist;
Ein Halteelement (4) zum Halten von mindestens einem aus dem Spritzenzylinder (2b) oder dem Behälter (3) derart, dass er entlang der Zylinderachsenrichtung des Spritzenzylinders (2b) beweglich ist, wobei die Spitze der Injektionsnadel (2a) dem Auslass des Behälters (3) zugewandt ist;
Eine Nadelkappe (2d), die aus einem Material, das von der Injektionsnadel (2a) durchstochen werden kann, ausgebildet ist, um die Injektionsnadel (2a) von der Spitzenseite zu bedecken, und die an einer Basisendseite an einem Basisabschnitt der Injektionsnadel (2a) oder an einer Außenfläche des Spritzenzylinders (2b) befestigt ist;
Einen Behälteradapter (8), der einen rohrförmigen Abschnitt (8a), der in engem Kontakt zu der Fläche des Dichtelements (3a) an einem Ende in der Rohrachsenrichtung befestigt ist, und einen flachen plattenförmigen Hauptkörperabschnitt, der die Öffnung auf der anderen Seite des rohrförmigen Abschnitts (8a) abdeckt, aufweist;
Der Hauptkörperabschnitt des Behälteradapters ist an einem dem Dichtelement (3a) zugewandten Abschnitt aus einem von der Injektionsnadel (2a) durchstechbaren Material ausgebildet, **gekennzeichnet dadurch, dass**
ein raumausbildender Körper (100), der einen der Fläche des Dichtelements zugewandten Raum mit einem zur Außenseite abgedichteten Raum ausbildet, von dem Behälteradapter (8) gebildet ist, und der raumausbildende Körper (100) an einem der Spitze der Injektionsnadel (2a) entlang der Zylinderachsenrichtung des Spritzenzylinders (2b) zugewandten Abschnitt aus einem Material ausgebildet ist, das von der Injektionsnadel (2a) durchstochen werden kann.

6. Mit einer Zweikomponentenmischung vorgefülltes Spritzenset wie in Anspruch 5 definiert, wobei:
Der Behälteradapter (8) auf der Außenseitenfläche der Dichtelementseite einen Flanschabschnitt aufweist, der in eine Richtung verläuft, welche die Zylinderachsenrichtung des Spritzenzylinders (2b) schneidet,
Die Nadelkappe (2d) auf der Außenseitenfläche der vorderen Seite einen Flanschabschnitt (2d4) aufweist, der in einer Richtung verläuft, welche die Zylinderachsenrichtung des Spritzenzylinders (2b) schneidet,
Das Halteelement (4) Folgendes aufweist: einen Montageabschnitt (50) zum Montieren des Spritzenzylinders (2b) und einen Schiebeabschnitt (4e), der, wenn er entlang der Zylinderachsenrichtung des Spritzenzylinders (2b) zu der Behälterseite bewegt wird, die zwei Flanschabschnitte (2d4) der Nadelkappe (2d) und des Behälteradapters (8) in einem überlagerten Zustand gegen den Behälter (3) schiebt, und den Verriegelungsteil, der den Zustand, in dem der Schiebeabschnitt (4e) die zwei Flanschabschnitte (2d4) gegen den Behälter (3) schiebt, hält.

7. Mit einer Zweikomponentenmischung vorgefülltes Spritzenset wie in Anspruch 5 oder 6 definiert, wobei:
Die Länge des rohrförmigen Abschnitts (8a) entlang der Rohrachsenrichtung geringer als 4 mm ist.

8. Mit einer Zweikomponentenmischung vorgefülltes Spritzenset wie in einem der Ansprüche 1 bis 7 definiert, wobei:
Ein Volumen eines von der Fläche des Dichtelements (3a) und des raumausbildenden Körpers (100) umgebenen Raumes in dem Bereich von 0,01 ml bis 0,03 ml liegt.

9. Mit einer Zweikomponentenmischung vorgefülltes Spritzenset wie in einem der Ansprüche 1, 4 oder 6 definiert, wobei:
es kennzeichnend ist, dass der Verriegelungsteil ein Klauenkörper ist, der mit einem konkaven Abschnitt oder einem Stufenabschnitt (3b1) auf der Außenfläche des Behälters (3) im Eingriff sein kann.

10. Mit einer Zweikomponentenmischung vorgefülltes Spritzenset wie in Anspruch 1 definiert, wobei:
ein aufnehmendes Gehäuse (1) umfasst, das die Gesamtheit aus dem Spritzenzylinder (2b), der Nadelkappe (2d), dem Behälter (3) und dem Halteelement (4) in einem nach der Außenseite hin abgedichteten Zustand aufnimmt.

11. Mit einer Zweikomponentenmischung vorgefülltes Spritzenset wie in Anspruch 3 bis 7 definiert, wobei:
ein aufnehmendes Gehäuse (1) umfasst, das die Gesamtheit aus dem Spritzenzylinder (2b), der Nadelkappe (2d), dem Behälteradapter (7, 8), dem Behälter (3) und dem Halteelement (4) in einem nach der Außenseite hin abgedichteten Zustand aufnimmt.

12. Mit einer Zweikomponentenmischung vorgefülltes Spritzenset wie in Anspruch 1 bis 11 definiert, wobei:
Die Spitze der Injektionsnadel (2a) von der Nadelkappe (2d) in einem nicht durchbohrten Durchstechungszustand gehalten wird.

## Revendications

1. Kit seringue de mélange de deux composants pré-rempli, comprenant :
un cylindre de seringue (2b) dans lequel une aiguille d'injection (2a) est fixée à une extrémité, un piston est inséré de manière coulissante depuis une ouverture à l'autre extrémité, et un composant solide ou un composant liquide constituant une solution d'injection y est introduit ;
un contenant (3) dans lequel une sortie est scellée avec un élément d'étanchéité (3a) qui peut être percé par l'aiguille d'injection (2a) et un composant liquide constituant la solution d'injection y est introduit ;
un élément de maintien (4) servant à maintenir au moins l'un du cylindre de seringue (2b) ou du contenant (3) de sorte qu'il soit mobile le long de la direction axiale de cylindre du cylindre de seringue (2b), la pointe de l'aiguille d'injection (2a) faisant face à la sortie du contenant (3) ;
un capuchon d'aiguille (2d) qui est formé d'un matériau qui peut être percé par l'aiguille d'injection (2a) pour couvrir l'aiguille d'injection (2a) depuis le côté pointe, et fixé au niveau d'un côté d'extrémité de base à une portion de base de l'aiguille d'injection (2a) ou à une surface externe du cylindre de seringue (2b) ; dans lequel un corps formant un espace (100) qui forme un espace faisant face à la surface de l'élément d'étanchéité (3a) avec un espace scellé vis-à-vis de l'extérieur est agencé de sorte à être en contact étroit avec la surface de l'élément d'étanchéité (3a), et le corps formant un espace (100) est formé d'un matériau qui peut être percé par l'aiguille d'injection (2a) au niveau d'une portion faisant face à la pointe de l'aiguille d'injection (2a) le long de la direction axiale de cylindre du cylindre de seringue (2b) ; et
dans lequel :
le capuchon d'aiguille (2d) présente une portion tubulaire (2d5) dans laquelle une direction axiale tubulaire se trouve le long de la direction vers l'élément d'étanchéité (3a) et un point de perçage prévu par l'aiguille d'injection (2a) est situé au niveau du côté interne, sur une portion avant faisant face à l'élément d'étanchéité (3a),
le corps formant un espace (100) est constitué par la portion avant du capuchon d'aiguille (2d) et la portion tubulaire (2d5) en contact étroit avec la surface de l'élément d'étanchéité (3a) ;
**caractérisé en ce que**
le capuchon d'aiguille (2d) présente une portion de rebord (2d4) étant dans une direction croisant la direction axiale de cylindre du cylindre de seringue (2b) sur la surface latérale externe de la portion avant ; et
l'élément de maintien (4) comprend une portion de montage (50) servant à monter le cylindre de seringue (2b), une portion de poussée (4e) servant à pousser la portion de rebord (2d4) contre le contenant (3) lorsqu'elle est déplacée vers le côté contenant le long de la direction axiale de cylindre du cylindre de seringue (2b), et une partie de verrouillage servant à maintenir un état dans lequel la portion de poussée (4e) pousse la portion de rebord (2d4) contre le contenant (3).

2. Kit seringue de mélange de deux composants pré-rempli selon la revendication 1, dans lequel :
la longueur de la portion tubulaire (2d5) le long de la direction axiale tubulaire est inférieure à 4 mm.

3. Kit seringue de mélange de deux composants pré-rempli, comprenant :
un cylindre de seringue (2b) dans lequel une aiguille d'injection (2a) est fixée à une extrémité, un piston est inséré de manière coulissante depuis une ouverture à l'autre extrémité, et un composant solide ou un composant liquide constituant une solution d'injection y est introduit ;
un contenant (3) dans lequel une sortie est scellée avec un élément d'étanchéité (3a) qui peut être percé par l'aiguille d'injection (2a) et un composant liquide constituant la solution d'injection y est introduit ;
un élément de maintien (4) servant à maintenir au moins l'un du cylindre de seringue (2b) ou du contenant (3) de sorte qu'il soit mobile le long de la direction axiale de cylindre du cylindre de seringue (2b), la pointe de l'aiguille d'injection (2a) faisant face à la sortie du contenant (3) ;
un capuchon d'aiguille (2d) qui est formé d'un matériau qui peut être percé par l'aiguille d'injection (2a) pour couvrir l'aiguille d'injection (2a) depuis le côté pointe, et fixé au niveau du côté d'extrémité de base à une portion de base de l'aiguille d'injection (2d) ou à la surface externe du cylindre de seringue (2b) ;
un adaptateur de contenant tubulaire (7) qui présente un axe de tube coïncidant avec la direction axiale de cylindre du cylindre de seringue (2b), et fixé en contact étroit avec la surface de l'élément d'étanchéité (3a) à une extrémité dans la direction axiale de tube ;
le capuchon d'aiguille (2d) présente une portion d'insertion (2d8) insérée le long de la direction axiale de tube de l'adaptateur de contenant depuis l'autre côté d'extrémité dans la direction axiale de tube de sorte à être en contact étroit avec la surface interne de l'adaptateur de contenant, au niveau d'une portion avant faisant face à l'élément d'étanchéité (3a) ;
**caractérisé en ce que**
un corps formant un espace (100) qui forme un espace faisant face à la surface de l'élément d'étanchéité avec un espace scellé vis-à-vis de l'extérieur est constitué par l'adaptateur de contenant (7) et la portion d'insertion (2d8) du capuchon d'aiguille (2d), et le corps formant un espace (100) est formé d'un matériau qui peut être percé par l'aiguille d'injection (2a) au niveau d'une portion faisant face à la pointe de l'aiguille d'injection (2a) le long de la direction axiale de cylindre du cylindre de seringue (2b).

4. Kit seringue de mélange de deux composants pré-rempli selon la revendication 3, dans lequel :
l'adaptateur de contenant (7) présente une portion de rebord (7b) étant dans une direction croisant la direction axiale de cylindre du cylindre de seringue (2b) sur la surface latérale externe du côté élément d'étanchéité,
l'élément de maintien (4) présente une portion de montage (50) servant à monter le cylindre de seringue (2b) et une portion de poussée (4e) servant à pousser la portion de rebord (7b) contre le contenant (3) lorsque le cylindre de seringue (2b) monté sur la portion de montage (50) est déplacé le long de la direction axiale de cylindre vers le côté contenant, et une partie de verrouillage servant à maintenir l'état dans lequel la portion de poussée (4e) pousse la portion de rebord (7b) contre le contenant (3).

5. Kit seringue de mélange de deux composants pré-rempli, comprenant :
un cylindre de seringue (2b) dans lequel une aiguille d'injection (2a) est fixée à une extrémité, un piston est inséré de manière coulissante depuis une ouverture à l'autre extrémité, et un composant solide ou un composant liquide constituant une solution d'injection y est introduit ;
un contenant (3) dans lequel une sortie est scellée avec un élément d'étanchéité (3a) qui peut être percé par l'aiguille d'injection (2a) et un composant liquide constituant la solution d'injection y est introduit ;
un élément de maintien (4) servant à maintenir au moins l'un du cylindre de seringue (2b) ou du contenant (3) de sorte qu'il soit mobile le long de la direction axiale de cylindre du cylindre de seringue (2b), la pointe de l'aiguille d'injection (2a) faisant face à la sortie du contenant (3) ;
un capuchon d'aiguille (2d) qui est formé d'un matériau qui peut être percé par l'aiguille d'injection (2a) pour couvrir l'aiguille d'injection (2a) depuis le côté pointe, et fixé au niveau d'un côté d'extrémité de base à une portion de base de l'aiguille d'injection (2a) ou à la surface externe du cylindre de seringue (2b) ;
un adaptateur de contenant (8) présentant une portion tubulaire (8a) qui est fixée en contact étroit avec la surface de l'élément d'étanchéité (3a) à une extrémité dans la direction axiale de tube, et une portion corps principal en forme de plaque plate qui couvre l'ouverture sur l'autre côté d'extrémité de la portion tubulaire (8a) ;
la portion de corps principal de l'adaptateur de contenant est formée d'un matériau qui peut être percé par l'aiguille d'injection (2a) au niveau d'une portion faisant face à l'élément d'étanchéité (3a), **caractérisé en ce que**
un corps formant un espace (100) qui forme un espace faisant face à la surface de l'élément d'étanchéité avec un espace scellé vis-à-vis de l'extérieur est constitué par l'adaptateur de contenant (8), et le corps formant un espace (100) est formé d'un matériau qui peut être percé par l'aiguille d'injection (2a) au niveau d'une portion faisant face à la pointe de l'aiguille d'injection (2a) le long de la direction axiale de cylindre du cylindre de seringue (2b).

6. Kit seringue de mélange de deux composants pré-rempli selon la revendication 5, dans lequel :
l'adaptateur de contenant (8) présente une portion de rebord étant dans une direction croisant la direction axiale de cylindre du cylindre de seringue (2b) sur la surface externe du côté élément d'étanchéité,
le capuchon d'aiguille (2d) présente une portion de rebord (2d4) étant dans une direction croisant la direction axiale de cylindre du cylindre de seringue (2b) sur la surface latérale externe du côté avant,
l'élément de maintien (4) comprend une portion de montage (50) servant à monter le cylindre de seringue (2b), et une portion de poussée (4e) qui pousse les deux portions de rebord (2d4) du capuchon d'aiguille (2d) et l'adaptateur de contenant (8) dans un état de superposition contre le contenant (3) lorsqu'elle se déplace vers le côté contenant le long de la direction axiale de cylindre du cylindre de seringue (2b) et la partie de verrouillage qui maintient l'état dans lequel la portion de poussée (4e) pousse les deux portions de rebord (2d4) contre le contenant (3).

7. Kit seringue de mélange de deux composants pré-rempli selon la revendication 5 ou 6, dans lequel :
la longueur de la portion tubulaire (8a) le long de la direction axiale de tube est inférieure à 4 mm.

8. Kit seringue de mélange de deux composants prérempli selon l'une quelconque des revendications 1 à 7, dans lequel :
un volume d'espace entouré par la surface de l'élément d'étanchéité (3a) et du corps formant un espace (100) est dans la plage de 0,01 ml à 0,03 ml.

9. Kit seringue de mélange de deux composants pré-rempli selon l'une quelconque des revendications 1, 4 ou 6, dans lequel :
il est **caractérisé en ce que** la partie de verrouillage est un corps de griffe qui peut être mis en prise avec une portion concave ou une portion étagée (3b1) sur la surface externe du contenant (3).

10. Kit seringue de mélange de deux composants pré-rempli selon la revendication 1, dans lequel :
il comprend un boîtier de logement (1) qui loge l'ensemble du cylindre de seringue (2b), le capuchon d'aiguille (2d), le contenant (3) et l'élément de maintien (4) dans un état scellé vis-à-vis de l'extérieur.

11. Kit seringue de mélange de deux composants pré-rempli selon l'une quelconque des revendications 3 à 7, dans lequel :
il comprend un boîtier de logement (1) qui loge l'ensemble du cylindre de seringue (2b), le capuchon d'aiguille (2d), l'adaptateur de contenant (7, 8), le contenant (3) et l'élément de maintien (4) dans un état scellé vis-à-vis de l'extérieur.

12. Kit seringue de mélange de deux composants pré-rempli selon l'une quelconque des revendications 1 à 11, dans lequel :
la pointe de l'aiguille d'injection (2a) est maintenue par le capuchon d'aiguille (2d) dans un état de perçage sans pénétration.
